# FASCICULE DE BREVET EUROPEEN

(11) **EP 2 285 923 B1**
(45) Date de publication et mention de la délivrance du brevet: **17.12.2014**
(21) Numéro de dépôt: 09726789.2
(22) Date de dépôt: 02.04.2009
(51) Int. Cl.: C09J 5/00

(54) **PROCEDE POUR ASSEMBLER DEUX SURFACES OU UNE SURFACE AVEC UNE MOLECULE D'INTERET**
VERFAHREN ZUM VERBINDEN VON ZWEI OBERFLÄCHEN ODER EINER OBERFLÄCHE MIT EINEM TEILGENOMMENEN MOLEKÜL
METHOD FOR ASSEMBLING TWO SURFACES, OR ONE SURFACE, WITH A MOLECULE OF INTEREST

(30) Priorité: 03.04.2008 FR 0852209; 27.10.2008 FR 0857269
(43) Date de publication de la demande: 23.02.2011
(73) Titulaire: Commissariat à l'Énergie Atomique et aux Énergies Alternatives, 75015 Paris (FR)
(72) Inventeur: BERTHELOT, Thomas, F-91940 Les Ulis (FR); DENIAU, Guy, F-78610 Auffargis (FR); HUC, Vincent, F-91190 Gif Sur Yvette (FR); LE, Xuan Tuan, Montreal, QC H2E 2C1 (CA); NEKELSON, Fabien, 78180 Montigny le Bretonneux (FR); ROUSSEL, Sébastien, F-91450 Soisy Sur Seine (FR); VIEL, Pascal, F-92190 Meudon (FR)
(74) Mandataire: Ilgart, Jean-Christophe
(86) Numéro de dépôt international: PCT/EP2009/053977
(87) Numéro de publication internationale: WO 2009/121944

(56) Documents cités:
- WO-A-03/080748
- US-A1- 2005 031 887

## Description

### DOMAINE TECHNIQUE

La présente invention concerne le domaine des surfaces pré-adhérentes et des procédés d'assemblage et de collage. Elle permet de réaliser un assemblage par contact direct entre deux matériaux ou entre un matériau et des molécules. Dans le cas de deux matériaux, le matériau de revêtement réagit par contact direct (sans « adhésif » au sens classique) sur le premier matériau.

L'invention est notamment utilisable pour coller ou immobiliser, sur une surface donnée, des nanotubes de carbone ou des graphènes isolés par exfoliation. La présente invention permet également l'immobilisation, sur une surface donnée, de particules métalliques, de polymères, de molécules organiques, de macromolécules et particulièrement de molécules biologiques. Dans ce dernier cas, la présente invention propose de nouveaux biocapteurs comprenant une surface adhérente telle qu'obtenue par le procédé de l'invention sur laquelle sont immobilisés un ou plusieurs éléments, identiques ou différents, choisis parmi les peptides, les polypeptides, les protéines comme les enzymes, les acides nucléiques, les anticorps ou fragments d'anticorps, les polysaccharides, les cellules et les fragments cellulaires.

L'invention est également relative à l'utilisation de différentes surfaces et notamment d'apprêts d'enduction convenablement sélectionnés afin de permettre l'assemblage, simple et reproductible, de différents matériaux ou molécules sur une surface enduite.

### ÉTAT DE LA TECHNIQUE ANTÉRIEURE

La micro-électronique s'intéresse à l'étude et à la fabrication de composants électroniques à l'échelle micrométrique. Ces composants sont fabriqués à partir de matériaux semi-conducteurs et minéraux, comme le silicium, au moyen de diverses technologies dont la photolithographie. Cette technologie permet l'intégration de nombreuses fonctions électroniques sur un même morceau de silicium (ou tout autre semi-conducteur) à plus bas-prix. Les circuits ainsi réalisés sont appelés « puces » ou « circuits intégrés ». Cependant, avec l'évolution des techniques de fabrication, la taille des composants continue de décroître. A l'échelle sub-micrométrique, des effets physiques parasites, autrefois sans importance, se trouvent grandement amplifiés. Les délais de transmission des signaux sont essentiellement dus aux capacités parasites d'interconnexion des éléments actifs et non au délai de traversée de ces mêmes éléments. L'objectif des chercheurs et ingénieurs est donc d'utiliser de nouvelles méthodes de conception pour limiter ces effets tout en améliorant la taille et le coût des composants, la vitesse de transmission et la consommation électrique.

Il est donc nécessaire de trouver de nouveaux matériaux et techniques de fabrication pour réduire la taille des composants et ainsi couvrir le domaine nanométrique. Ce domaine est celui des molécules organiques. La principale illustration d'électronique moléculaire est le nanotube de carbone. Ce matériau est, depuis quelques années, à l'origine d'un nombre considérable de publications scientifiques. Le nanotube de carbone est une structure cristalline particulière, de forme tubulaire, creuse et parfois close, composée d'atomes de carbone disposés régulièrement en réseaux hexagonaux.

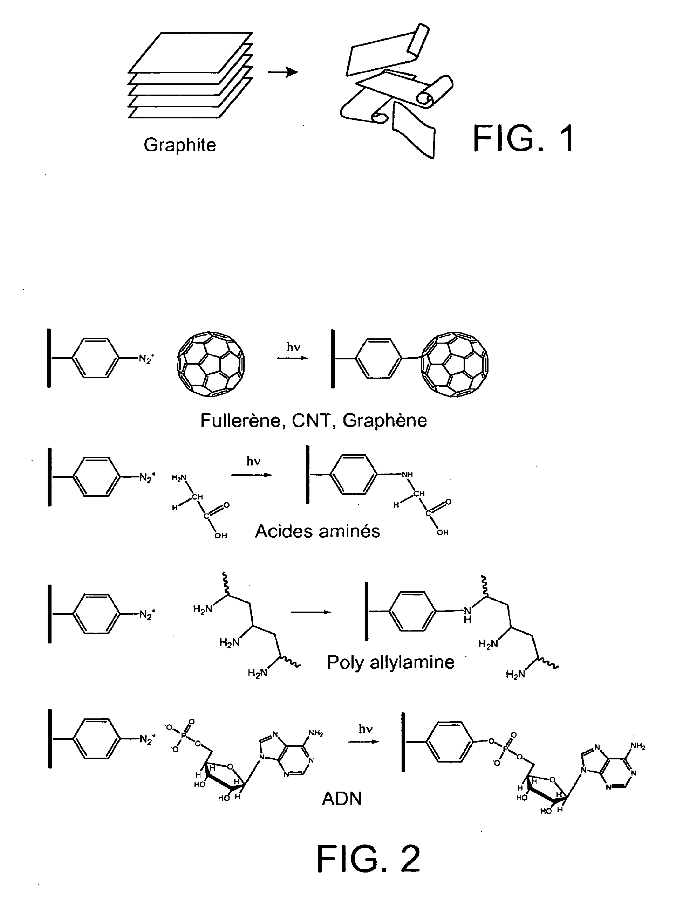

Les nanotubes de carbone permettent de réaliser des transistors à un niveau de miniaturisation jamais atteint jusqu'à présent. Malheureusement, lors de leur fabrication, on obtient à la fois des nanotubes conducteurs et des nanotubes semi-conducteurs alors que seuls ces derniers possèdent des propriétés intéressantes. La nécessité d'un triage parmi les nanotubes obtenus afin de récupérer les nanotubes semi-conducteurs a fait apparaître une alternative aux nanotubes de carbone. Il s'agit de l'utilisation de mono-feuillets de graphite ou graphènes. Cette nouvelle voie est en pleine expansion. Un graphène est généralement décrit comme un nanotube ouvert. Le graphène se trouve être le composant unique du graphite hautement orienté ou HOPG (pour « High Organized Phase Graphite ») que l'on peut représenter comme un millefeuille de graphènes. La difficulté consistant à réussir à obtenir un graphène à partir d'un bloc d'HOPG ou de graphite contenant plusieurs milliers de feuillets. La technique la plus utilisée consistant à effeuiller ou exfolier le bloc d'HOPG ou le graphite par clivage adhésif est représentée à la Figure 1.

Le graphène est particulièrement intéressant parce qu'il est conducteur selon une direction et semi-conducteur dans une autre. Tous les graphènes sont donc potentiellement utilisables. Il ne reste alors que les connexions électriques à mettre en place selon le bon axe. Ce processus est déjà maîtrisé. Cependant, le graphène doit être immobilisé sur une surface pour être utilisé. Actuellement, on utilise pour cette immobilisation des adhésifs commerciaux. Ces procédures conduisent à la formation de couches adhésives d'épaisseurs variables et mal contrôlées. Au-delà, les contraintes mécaniques fortes rencontrées lors du processus d'exfoliation se traduisent souvent par des déchirures ou des plis au sein des feuillets minces de graphène. Un décrochage complet de la surface est également possible. De plus, l'adhésion des graphènes est réalisée au travers de mécanismes de physisorption eux-mêmes mal contrôlés.

Un adhésif se définit comme un produit capable de maintenir ensemble des matériaux, notamment par adhérence. L'adhérence est ainsi l'état dans lequel deux surfaces sont maintenues assemblées par des forces interfaciales. Les adhésifs sont obtenus par formulation de plusieurs constituants qui apportent, chacun, une ou plusieurs fonctions technologiques, la résine de base conduisant à la dénomination de l'adhésif.

Les conditions de mise en oeuvre de l'adhésif (rhéologie, mouillabilité des surfaces...) et les performances finales de l'assemblage sont notamment liées aux caractéristiques intrinsèques de l'adhésif mais aussi aux adjuvants de formulation tels que plastifiants, tensioactifs, charges, etc... ; à la conception de l'assemblage (géométrie des joints) ; aux préparations des surfaces et aux paramètres de mise en oeuvre. Il n'y a pas d'adhésif universel, mais plusieurs dizaines de familles d'adhésifs.

Pour répondre à ce problème, l'utilisation de l'électrogreffage a été proposée dans la demande internationale WO 03/080748. Le procédé permet la fixation de macro-objets sur une surface conductrice ou semi-conductrice de l'électricité par électro-greffage à partir d'une solution électrolytique comprenant, sous forme dissoute, particulaire ou émulsionnée, au moins un macro-objet constitué d'une partie macrostructurale fonctionnalisée par au moins un groupement électro-actif et apte à provoquer l'électro-greffage dudit macro-objet sur la surface. Le greffage est réalisé par électrolyse de la solution en utilisant la surface conductrice ou semi-conductrice à recouvrir comme électrode de travail pour conduire, par électro-réduction ou électro-oxydation de ladite solution, à un revêtement greffé du macro-objet ou de ses produits de condensation sur ladite surface. Cette méthode reste cependant limitée aux surfaces conductrices ou semiconductrices de l'électricité et elle nécessite en outre la présence de groupes spécifiques à la surface des objets à greffer.

Dans le domaine de la microélectronique, les silanes sont habituellement utilisés pour modifier une surface au travers d'un greffage chimique de molécules bifonctionnelles qui conduit à des liaisons fortes entre la surface et une entité. Cependant, la réactivité chimique de ces composés est faible et nécessite des recuits dépassant souvent les 100°C pour immobiliser, d'abord, les silanes sur la surface avec la formation d'un lien siloxane qui reste cependant facilement hydrolysable et, ultérieurement, le revêtement.

Des diazo-résines ont été employées pour former des couches multiples d'enzymes ou de polyoxométallates à la surface de différents matériaux par complexation électronique puis photoréaction [Materials Letters, 58, 2004, 3441-3446 et Electrochim. Acta. 49, 2004, 4777-4786]. Ce procédé reste limité car il nécessite l'existence d'interactions électroniques entre les entités destinées à être déposées à la surface et les diazo-résines. En outre, il ne semble pas permettre un greffage avec la surface.

Dans le domaine des biocapteurs, une grande variété de systèmes analytiques ont été mis au point. Ces biocapteurs peuvent utiliser des enzymes, des protéines, des anticorps, des acides nucléiques, des polysaccharides ou des cellules vivantes, en combinaison avec des transducteurs électrochimiques, optiques, piézoélectriques, magnétiques ou thermiques. Les biocapteurs sont des instruments de mesure visant à accomplir la détection d'une molécule dans un échantillon souvent complexe. La phase cruciale de reconnaissance moléculaire est réalisée grâce au biorécepteur, le plus souvent un élément biologique immobilisé, formant la couche sensible. Lors de la reconnaissance moléculaire, la présence de la molécule cible se traduit directement ou indirectement par un signal. Celui-ci peut être une émission de photons, l'apparition de produit(s) de réaction, ou une variation de masse, de pH ou de propriétés électriques. La seconde phase, dite de transduction/amplification a pour rôle de collecter et de traduire ce signal en un courant électrique mesurable. Dès lors, les données peuvent être acquises et traitées sous forme de valeurs numériques ; il s'agit de la troisième phase d'acquisition et de traitement des données. Le succès du fonctionnement d'un biocapteur est étroitement lié à la bonne synergie entre les différents éléments le composant : le support, la méthode d'immobilisation et le système de détection/transduction.

Le terme générique de « biopuce » regroupe un ensemble de dispositifs analytiques, dont les technologies sont en partie issues des développements réalisés pour les biocapteurs. Les biopuces comportent plusieurs éléments de reconnaissance, généralement disposés côte-à-côte sur un support donné, avec une densité plus ou moins élevée. Ainsi, une faible quantité de biomolécules est immobilisée sur une surface de petite taille (quelques millimètres à quelques centimètres), permettant de réaliser des mesures sur de faibles volumes d'échantillons (quelques nanolitres à quelques microlitres). En effet, le principe de fonctionnement des biopuces repose sur celui des biocapteurs, avec en plus de l'approche intégrée du système, des notions de miniaturisation et de parallélisation. Généralement, les éléments biologiques sont disposés sous forme de plots, agencés en parallèle à la surface d'un support. Il existe différentes catégories de biopuces dont les biopuces à ADN et les biopuces à protéines, complétées par les biopuces à peptides et à sucres. Au sein de chaque catégorie, l'application recherchée peut être différente. Il peut être envisagé de détecter de manière quantitative la présence d'une molécule cible (dosage) dans un échantillon complexe, ou d'étudier les interactions d'une biomolécule sur un ensemble de molécules immobilisées sous forme de plots (criblage).

Le support ainsi que la nature des biomolécules à fixer conditionnent la méthode d'immobilisation à employer. Généralement, le choix du support est lui-même conditionné par la méthode de détection qui sera utilisée. On peut ainsi distinguer les supports « actifs » et les supports « passifs », vis-à-vis de la détection. Les supports dits « actifs » sont des matériaux conducteurs, utilisés pour des détections et/ou des immobilisations électrochimiques (ampérométrie, impédance, électrochimiluminescence), ou optiquement actifs (CCD, [Mallard et al., 2005, Biosensors & Bioelectronics, 20, 1813-1820]).

Les méthodes d'immobilisation déjà décrites et validées peuvent être classées selon la nature des interactions mises en jeu. Il existe (i) les immobilisations sans greffage covalent impliquant une adsorption, (ii) le piégeage dans un gel ou un polymère, impliquant des liaisons de faible énergie (de type hydrophobe, électrostatique, ou de rétention mécanique dans un réseau), (iii) des immobilisations dites « covalentes », où des réactions chimiques dites « de couplage » conduisent à la formation de liaisons covalentes entre biomolécules et support.

L'adsorption est la méthode la plus simple pour immobiliser des éléments biologiques. Elle met en jeu des interactions de faible énergie entre le support et les biomolécules. La capacité d'adsorption de nombreux supports modifiés en surface par des composés tels que le méthyl-silane, le polydiméthylsiloxane, le polystyrène, le polyimide, le thio-silane, l'époxy-silane, le polyéthylèneglycol-silane, l'amino-silane, l'agarose et les alcanethiols est fonction du degré d'hydrophobicité des protéines à immobiliser. D'autres matériaux peuvent être également directement utilisés pour l'immobilisation par adsorption tels que le polyfluorure de vinylidène, l'acrylonitrile-butadiène-styrène, le polydiméthylsiloxane, le nylon, le nylon chargé, la nitrocellulose, le silicium nano-poreux. L'utilisation de support dont la surface à été préalablement modifiée par de la poly-lysine, de l'hydrochlorure de poly(allylamine), du poly(styrènesulfonate) de sodium, permet l'adsorption par l'intermédiaire de forces électrostatiques.

Ces méthodes de piégeage comprenant l'encapsulation, la rétention dans un gel ou un polymère ou la co-réticulation sont mises en oeuvre de diverses manières. Les polymères utilisés peuvent être des dérivés polyvinyliques, des polyéthylèneglycols, du dextran ou de l'aminodextran. Ils peuvent être photopolymérisables, ou encore électropolymérisables comme le polypyrrole, le polythiophène ou la polyaniline. La co-réticulation est en général réalisée entre une protéine d'intérêt et une protéine neutre, le plus souvent la BSA (albumine de sérum bovin), et en présence d'un agent pontant, généralement le glutaraldéhyde. Les méthodes de piégeage permettent d'une manière générale d'augmenter la charge en protéine par unité de surface.

L'immobilisation par affinité utilise les propriétés naturelles de reconnaissance et d'interaction de certaines molécules. Le couple streptavidine/biotine est majoritairement employé dans ce dernier type d'immobilisation non-covalente compte tenu de la très forte affinité entre ces deux entités (Kd = 10⁻¹⁵ M). Cette méthode consiste soit en l'immobilisation de streptavidine (protéine homotétramérique) soit de biotine à la surface d'un support. L'immobilisation a ensuite lieu indirectement grâce à la mise en présence de protéine ou une autre molécule préalablement marquée soit par la biotine soit par la streptavidine. Il est également possible d'utiliser la forte affinité de ce couple streptavidine/biotine afin d'immobiliser des particules de grande taille (billes de polystyrène de diamètre : 1 µm). Cette dernière technique permet, grâce aux billes-streptavidine fixées sur un support activé par de la biotine, d'augmenter la quantité de matériel biologique immobilisé par unité de surface. D'autres protéines telles que les protéines A et G peuvent être utilisées pour leur propriété d'interaction spécifique avec des anticorps par exemple. Ce type d'agents biologiques sont généralement adsorbés à la surface de polydiméthylsiloxane ou par couplage préalable avec du glutaraldéhyde dans différents polymères hydrophiles (polyéthylenimine, dextran, polyvinyl alcool, aminodextran et 3-aminopropyltriéthoxysilane).

Des méthodes chimiques de modification de surfaces sont utilisées pour fixer de manière covalente des protéines et des acides nucléiques. Dans ce cas, le support doit présenter en surface des groupements chimiques réactifs tels que des groupements hydroxyle, amine, aldéhyde, époxyde, carboxyle, azide, alkyne, hydrazone ou des thiols afin de réaliser des réactions de couplage. Les méthodes conduisant à un couplage covalent entre le support et les biomolécules dépendent essentiellement de la nature du substrat employé. Les différentes méthodes sont présentées en fonction du matériau utilisé comme support.

Le verre est couramment modifié en utilisant la chimie des silanes. Les méthodes conduisant à la formation de monocouches auto-assemblées (SAM, *Self Assembled Monolayers*) sont privilégiées. Ces techniques consistent à générer une monocouche de composés alkylsilanes, fonctionnalisant les surfaces de verres. Ces silanes présentent à leur extrémité des fonctions permettant la création d'une liaison covalente avec l'agent biologique tels qu'une fonction amine, acide carboxylique, aldéhyde, hydroxyle, thiol, maléimide ou ester de succinimide.

Parmi les métaux, le platine peut être préalablement oxydé, avant d'être modifié chimiquement par des aminosilanes.

Afin d'utiliser des surfaces d'or, les méthodes conduisant à la formation de monocouches auto-assemblées (SAM pour « Self Assembled Monolayers ») sont privilégiées. Ces techniques consistent à générer une monocouche de composés thiolés, fonctionnalisant les surfaces d'or. La surface d'or fonctionnalisée présente alors des propriétés qui dépendent de la longueur de la chaine et des fonctions accessibles à leur extrémité. Ainsi, les lipides déposés sont choisis en fonction des propriétés recherchées : immobilisation par adsorption, par couplage covalent ou pour une transduction électrochimique. Pour le développement de supports destinés à des mesures de SPR, deux technologies sont majoritairement utilisées : la chimie des thiols seule, et la chimie des thiols supportant des polymères de type dextran. Dans le cas de la chimie des thiols, la surface d'or est traitée chimiquement afin d'être fonctionnalisée par une monocouche d'amine-alcenethiol, utilisée ensuite afin de fixer covalemment les biomolécules sur le support. Cette technologie, a permis de développer des biopuces pour la détection en imagerie par SPR (SPRi) utilisant des oligonucléotides immobilisés, des protéines de faible poids moléculaire immobilisées et des carbohydrates immobilisés. L'immobilisation impliquant des polymères de type dextran est également très utilisée pour l'immobilisation de biomolécules sur des supports d'or pour la SPR. Cette technologie est celle utilisée par la société Biacore®. Cette technique consiste à utiliser des polymères dextran aminés, ou oxydés (acide carboxylique) afin de fixer les biomolécules par couplage covalent sur ceux-ci.

Les surfaces à base de carbone, et plus particulièrement le carbone vitreux, peuvent être oxydés électrochimiquement, afin de faire apparaître en surface des fonctions oxygénées réactives. Les conditions requises sont un milieu acide, et un potentiel oxydant (+1,6 V vs-SCE). Les fonctions carboxyliques qui apparaissent en surface peuvent alors être impliquées dans des réactions de couplage de type carbonyl/amine primaire, avec notamment des agents pontants : éthylènediamine seul ou éthylènediamine puis glutaraldéhyde. Ces procédés ont été utilisés pour l'immobilisation de brins d'ADN, de sondes oligonucléotidiques et d'enzymes.

La société Affymetrix® utilise une technique de photolithographie permettant la synthèse *in situ* de sondes nucléotidiques fixées covalemment sur la surface d'une puce en quartz (1 x 1 cm). Pour cela, des étapes de réaction chimique de couplage s'enchaînent, avec des étapes de déprotection. Ces étapes de déprotection ciblent des zones de la puce grâce à une technique de photolithographie : un masque portant les ouvertures appropriées permet de déprotéger les oligonucléotides choisis en vue du couplage. Cette méthode permet de créer des puces supportant jusqu'à 1,3 million de brins (au total), pouvant représenter jusqu'à 10 000 plots de séquences différentes.

Les supports de type polydiméthylsiloxane (PDMS) peuvent également être utilisés pour des immobilisations covalentes. La chimie utilisée met en oeuvre les mêmes procédés que ceux employés sur le verre. Il s'agit de la chimie des silanes appliquée au PDMS, après des étapes d'oxydation, soit par la méthode des plasmas d'oxygène, soit par la méthode utilisant du peroxyde d'hydrogène en milieu acide. Ce type de support modifié a pu être fonctionnalisé enfin par une couche de biotine. Le PDMS après oxydation en milieu acide et silanisation, a permis le greffage covalent de molécules telles que des protéines, des peptides ou des oligonucléotides.

Les techniques dites « d'électro-adressage » de biomolécules sont des méthodes d'immobilisation récentes. Tout d'abord, les biomolécules sont modifiées avec une unité électropolymérisable. En appliquant un potentiel électrochimique aux bornes d'un microréseau sérigraphié par une électrode de platine, les biomolécules modifiées sont électro-adressées aux bornes.

Historiquement, l'immobilisation par électro-adressage est une technologie récente qui est apparue avec les recherches réalisées sur les électropolymères conducteurs. Par définition, ceux-ci sont des structures polymériques réalisées par électroréduction ou électro-oxydation à partir de monomères en solution. Les films polymérisés ont des propriétés conductrices qui proviennent d'une forte mobilité des électrons, due à la conjugaison des liaisons C=C, le long de toute la chaine polymérisée. Le premier biocapteur utilisant des films de polypyrrole reposait sur le piégeage d'une enzyme, durant le processus d'électropolymérisation du pyrrole en solution. Cette technique exploite la capacité du pyrrole et/ou de ses dérivés à former des films insolubles, adsorbés à la surface de l'électrode. Dans ce type de système, le polymère joue non-seulement le rôle de matrice d'immobilisation de l'enzyme, mais permet également un transfert électronique rapide depuis l'enzyme jusqu'à l'électrode. Actuellement, les systèmes d'immobilisation utilisant ces électropolymères sont essentiellement associés à des détections ampérométriques. Les polymères utilisés sont de différentes natures : polyaniline, polypyrrole, polyacétylène, polyphénylène ou polythiophène.

Parmi ces systèmes, nous pouvons distinguer deux classes majeures d'immobilisation impliquant des électropolymères. D'une part, le piégeage « mécanique » de biomolécules durant le processus d'électropolymérisation, qui est efficace pour les molécules de haut poids moléculaire telles que les protéines et, d'autre part la co-polymérisation de monomères de type pyrrole avec des biomolécules fonctionnalisées par des unités pyrroles. Cette dernière est plus efficace pour immobiliser des biomolécules de « petite taille » telles que des oligonucléotides ou des polypeptides, mais peut également être utilisée pour des molécules de haut poids moléculaire.

Des enzymes de type oxydase ont été immobilisées par cette technique dans des films d'électropolymère conducteurs : polyaniline, polyindole, polypyrrole et poly(ophenyldiamine). D'autres enzymes ont également été immobilisées par électro-adressage, notamment une invertase dans un film mixte de polypyrrole/PMMA-co-PMMT (polymethyl-methacrylate-copolymethyl- thienyl-methacrylate). De manière comparable, une tyrosinase a été immobilisée par piégeage dans un film électrogénéré de polythiophène à la surface d'une électrode de carbone vitreux. Cette méthode de piégeage de biomolécules lors de l'électropolymérisation a également été décrite pour la réalisation d'immunocapteurs. Plus récemment, ce type d'immobilisation fut employé pour réaliser des plots de plusieurs anticorps électro-adressés dans du polypyrrole sur une surface de microélectrodes interdigitées.

Cette seconde méthode d'immobilisation impliquant également ces électropolymères repose sur la co-électropolymérisation de monomères et de biomolécules fonctionnalisées par un dérivé de ce monomère. Ce procédé est plus couramment utilisé pour les molécules de petite taille, et conduit à une fixation covalente des biomolécules au sein du polymère, ce qui évite les problèmes de relargage (désorption...). Ainsi, des acides-aminés et des di-peptides fonctionnalisés par du pyrrole ont été coélectropolymérisés dans des films de pyrrole. Utilisant la même approche de co-électropolymérisation, un dérivé de biotine a été électro-adressé dans un film de polydicarbazole à la surface d'une électrode de carbone vitreux. Ce support permet ensuite d'immobiliser un complexe avidinepolyphénol oxydase, et ainsi de détecter par ampérométrie la L- et la D-noradrénaline. D'autre part, un réseau de 48 électrodes d'or de 50 µm x 50 µm chacune a été utilisé comme base pour l'électro-adressage d'oligonucléotides et de pyrrole co-polymérisés, utilisant des brins modifiés en 5' par du pyrrole. Cette méthode directe d'immobilisation fut aussi mise en oeuvre pour la réalisation d'une biopuce à ADN. Basées sur ce modèle d'immobilisation par co-électropolymérisation de dérivés pyrrolés, des microélectrodes modifiées par des polypeptides ont également été obtenues. Cette méthode d'immobilisation par électro-adressage a également été employée pour créer des plots sur des supports en or, pour des mesures en imagerie de résonnance des plasmons de surface (SPRi). Ainsi, des dérivés pyrrolés d'oligonucléotides ont été co-électropolymérisés à la surface d'une puce d'or pour SPRi de 1 cm². L'étape de déposition des différentes couches sensibles s'effectue alors grâce à un système de « pin-electrospotting ». La même approche a également été développée pour l'immobilisation de protéines.

Ces systèmes d'électro-adressage par « pin-electrospotting » sur or ont permis de former des plots de films insolubles de pyrrole-proteines, pyrrole-oligonucléotides et pyrrole-peptides, permettant d'effectuer des mesures en SPRi avec des performances équivalentes aux systèmes classiques d'immobilisation. Une méthode originale d'électro-adressage indirect sur or utilise un réseau d'électrodes en or fonctionnalisées par une monocouche exposant des fonctions mono-ester d'hydroquinone. Le greffage d'amino-biotine en solution sur ces fonctions est déclenché grâce à une électro-oxydation. Un réseau d'électrodes sérigraphié a également été utilisé afin de mettre au point une méthode d'immobilisation de protéines adressées électrochimiquement. Ainsi la palette de couches sensibles pouvant être obtenues grâce à des films électropolymérisés est assez large, et peut s'appliquer à l'immobilisation de protéines, d'anticorps ou d'oligonucléotides. Les biocapteurs/biopuces ainsi réalisés permettent aussi bien des détections optiques qu'électrochimiques.

L'immobilisation par électro-adressage de biomolécules reposant sur l'électro-réduction des sels de diazonium n'est, à l'heure actuelle, que très peu développée. Ce procédé repose sur les propriétés particulières de greffage des sels d'aryl-diazonium introduit par [Delamar; M. et al., 1992, Journal of The American Chemical Society, 114, 5883-5884] :
(a) Les sels de diazonium peuvent être formés à partir de dérivés de l'aniline, dans une solution acide de NaNO₂ ;
(b) ce sel de diazonium peut alors être électroréduit pour conduire à la libération d'azote, et à la formation d'un radical aryle de forte réactivité ;
(c) ce dernier se greffe, sur la surface de l'électrode qui a fournit l'électron nécessaire à son électro-réduction. Il se forme une liaison covalente de type C-X, où X peut être de l'or, du cobalt, du nickel, du zinc, de l'ITO (film d'oxyde d'indium-étain), du platine, du cuivre, du graphite, du diamant, ou du silicium. Récemment, un type de couche activée a été obtenu par électrodéposition de sels de diazonium préalablement modifiés par un groupement maléimide. La couche ainsi obtenue peut être par la suite fonctionnalisée par des molécules ou des agents biologiques contenant une fonction thiol libre.

L'électro-adressage de sels de diazonium a été mise en oeuvre pour la première fois sur des électrodes de carbone vitreux, afin d'immobiliser de manière indirecte une enzyme. La réaction d'électro-greffage était alors utilisée afin de fonctionnaliser l'ensemble de la surface de carbone vitreux et ainsi d'obtenir une couche d'acétate de phényle. Cette méthode a été utilisée pour dérivatiser la surface afin de réaliser un greffage covalent par couplage chimique de la glucose oxydase. Il s'agit donc ici d'une méthode indirecte d'électro-adressage. La réaction de diazotation sur un dérivé aniline a été également utilisée afin de greffer une monocouche de biotine. Un conjugué biotine-aniline a alors été diazoté pour former un dérivé biotine-aryldiazonium, puis greffé par électro-réduction à la surface d'une électrode de carbone sérigraphiée. Ainsi, la surface de l'électrode devient un point d'ancrage covalent pour la streptavidine. Cette surface va permettre la fixation de la phosphatase alcaline biotinylée. Une méthode d'électro-adressage direct pour la peroxydase du raifort (HRP) a été développée. Un couplage entre le 4-carboxyphényldiazonium et la HRP, via un carbodiimide est réalisé, puis l'adduit HRP-aryldiazonium est électro-adressé sur une électrode de carbone vitreux. Il en résulte la formation d'une couche sensible d'HRP fixée covalemment, permettant de détecter le peroxyde d'hydrogène par voltammétrie cyclique. Il est envisageable d'étendre cette méthode à une plus large variété de supports, sur lesquels il a été montré que les aryl-diazoniums pouvaient se greffer tels que le fer, le platine, le cobalt, le nickel, le zinc, le cuivre, l'or, l'ITO et le silicium. Récemment, un sel de diazonium préalablement modifié par un groupe maléimide a été fonctionnalisé avec un ADN_{SS} [Harper, J.C. et al., 2008, Langmuir, 24, 2206-2211] puis a été électro-adressé avec succès.

Le principal inconvénient des immobilisations par adsorption est la désorption quasi systématique et non contrôlée des biomolécules. En effet, les énergies d'interactions entre les molécules et le support sont sensibles aux variations de pH et de force ionique du milieu. Ce phénomène peut, par exemple, intervenir lors de dosages réalisés dans des milieux complexes tels que des échantillons naturels.

L'immobilisation covalente d'objets biologiques ou de molécules biologiquement actives passe soit par l'activation d'une surface préalablement modifiée, soit par la modification de l'objet biologique ou de la molécule biologiquement active avant son immobilisation sur le support. Ces étapes d'activation sont généralement effectuées dans des milieux incompatibles avec les milieux biologiques et entraînent la formation de produits secondaires pouvant entraîner des biais lors de la mesure. Généralement, de nombreuses et couteuses étapes de purification sont obligatoires pour obtenir une biopuce opérationnelle. Les modifications d'objets biologiques ou de molécules biologiquement actives peuvent entraîner une perte d'activité de ces dernières. De plus, leurs modifications ne s'effectuent généralement pas de façon contrôlées et leur étude reste longue et coûteuse surtout en présence d'un nombre important d'objets à modifier. Le type de stratégie pour l'immobilisation covalente est également dépendant du substrat ou support utilisé pour la biopuce et pour le mode de détection envisagé.

Il existe donc un réel besoin d'une méthode d'adhésion efficace entre une surface de tout type et une entité comme une autre surface, telle que celle de graphène ou de nanotubes de carbone, ou une molécule telle qu'une molécule utilisable dans les biocapteurs et ce sur la base de liaisons de type covalent.

### EXPOSÉ DE L'INVENTION

La présente invention permet de résoudre les problèmes techniques et inconvénients listés ci-dessus puisqu'elle concerne des surfaces apprêtées, leur procédé de préparation ainsi que leur utilisation avec toute autre surface, permettant ainsi de réaliser l'adhésion entre deux matériaux dont l'un présente une surface réactive ou entre une telle surface réactive et des molécules d'intérêt en vue de leur immobilisation.

Plus précisément, la présente invention concerne un procédé d'assemblage d'au moins une zone d'une première surface avec au moins une zone d'une seconde surface ou avec une molécule d'intérêt, comprenant une étape consistant à mettre en contact ladite zone de ladite première surface avec ladite zone de ladite seconde surface ou avec ladite molécule d'intérêt, ladite zone de ladite première surface présentant au moins une entité radicalaire et/ou ionique.

La première surface peut être désignée, dans le cadre de la présente invention, par l'expression « surface réactive ». Ainsi, les expressions « première surface » et « surface réactive » sont des expressions équivalentes.

La seconde surface peut être désignée, dans le cadre de la présente invention, par l'expression « surface de revêtement ». Ainsi, les expressions « seconde surface » et « surface de revêtement » sont des expressions équivalentes.

Au sens de la présente invention, « l'assemblage » correspond à une méthode d'assemblage chimique de deux entités à l'issue de laquelle lesdites entités sont maintenues assemblées par des interactions chimiques fortes. Cet assemblage ou adhésion correspond généralement à la formation de liaisons covalentes, c'est-à-dire la mise en commun ou le transfert d'électrons entre atomes appartenant aux deux entités.

Avantageusement, le procédé d'assemblage selon l'invention comprend les étapes successives suivantes :
a) éventuellement soumettre une zone de ladite première surface présentant au moins un précurseur d'un primaire d'adhésion, à des conditions adéquates permettant d'obtenir, à partir dudit précurseur d'un primaire d'adhésion, au moins un primaire d'adhésion,
b) soumettre ladite zone de ladite première surface présentant au moins un primaire d'adhésion, éventuellement obtenu à l'étape (a), à des conditions non-électrochimiques pour obtenir, sur ladite zone, au moins une entité radicalaire et/ou ionique ;
c) mettre en contact ladite zone de ladite première surface présentant au moins une entité radicalaire et/ou ionique obtenue à l'étape (b), avec ladite zone de ladite seconde surface ou avec ladite molécule d'intérêt.

Le terme « molécule d'intérêt » désigne, dans le cadre de la présente invention, toute molécule, et plus particulièrement toute molécule de nature organique, susceptible de réagir avec une entité radicalaire ou avec une entité ionique. A titre d'exemples de molécules d'intérêt utilisables dans le cadre de la présente invention, on peut citer notamment :
- les molécules organiques comportant des bases organiques faibles comme CO₂⁻, SO₃²⁻, les amines et les molécules aromatiques azotées ;
- les macromolécules organiques comme les porphyrines, les phtalocyanines et les dendrimères ;
- les molécules biologiques comme les peptides, les protéines telles que les enzymes, les anticorps ou fragments d'anticorps, les récepteurs cellulaires ou membranaires, les polysaccharides, les cellules ou parties cellulaires telles que des organites ou des membranes cellulaires et les acides nucléiques tels que ADN et ARN ;
- une molécule hydrophobe telle que définie ci-après.

La Figure 2 présente certaines de ces molécules d'intérêt et leur mise en oeuvre dans le cadre du procédé selon l'invention.

Au sens de la présente invention, par « surface » il faut entendre la partie extérieure d'un corps ou d'un support solide, qui le limite en tout sens. Dans la mesure où pour un même corps (ou même support solide), on peut définir conceptuellement différentes surfaces, la surface réactive et la surface de revêtement peuvent bien entendu appartenir à un même corps (ou support solide) ou à deux corps (ou supports solides) différents. L'invention s'applique à tout type de surface quelle que soit sa géométrie. Cette dernière peut être simple, comme une surface parfaitement plane, ou complexe, comme une surface rugueuse, ou présentant des cavités non obstruées et ce quel que soit le matériau constituant la surface et le reste du corps ou du support solide dont elle dépend.

La taille de la seconde surface est variable. Elle varie généralement entre l'échelle micrométrique et nanométrique. Ainsi il peut s'agir de surfaces de corps de taille nanométrique, ou nano-objets (NB), comme la surface de nanoparticules (NP), de nanotubes de carbone (CNT) simple paroi (SWCNT) ou multi parois (MWCNT), de feuillets de graphène ou de nanofils de silicium, ou de surfaces de taille micrométrique comme la surface de biopuces telles qu'utilisées dans l'industrie ou de particules métalliques.

L'invention est applicable à une grande diversité de surfaces d'intérêt (première et seconde surfaces) dont la composition peut-être choisie parmi une grande variété de matériaux car le procédé met à profit un mécanisme d'assemblage de nature ionique et/ou radicalaire et généralement de nature radicalaire. Ainsi, les première et seconde surfaces peuvent être de nature organique ou non organique, ou de nature composite avec éventuellement une composition non uniforme.

Toute surface présentant un ou plusieurs atome(s) ou groupement(s) d'atomes pouvant être impliqué(s) dans une réaction d'addition ou de substitution radicalaire, tel que CH, les carbonyles (cétone, ester, acide, aldéhyde), OH, SH, les éthers, les amines, les halogènes, comme F, Cl, Br, est notamment concernée par la présente invention.

Les surfaces de nature inorganique peuvent être notamment choisies parmi les matériaux conducteurs comme les métaux, les métaux nobles, les métaux oxydés, les métaux de transition, les alliages métalliques et par exemple Ni, Zn, Au, Pt, Ti ou l'acier. Il peut également s'agir de matériaux semi-conducteurs comme Si, SiC, AsGa, Ga, etc. Il est également possible d'appliquer le procédé à des surfaces non conductrices comme les oxydes non conducteurs tels que SiO₂, Al₂O₃ et MgO. De manière plus générale, une surface inorganique peut être constituée, par exemple, d'un matériau amorphe, tel qu'un verre contenant généralement des silicates ou encore une céramique, aussi bien que cristallin comme le diamant, du graphite pouvant être plus ou moins organisé, comme du graphène, du graphite hautement orienté (HOPG), ou des nanotubes de carbone.

A titre de surface de nature organique, on peut citer notamment des polymères naturels comme le latex ou le caoutchouc, ou artificiels comme les dérivés de polyamide ou de polyéthylène, et notamment les polymères présentant des liaisons de type n comme les polymères portant des liaisons éthyléniques, des groupements carbonyles, imine. Il est également possible d'appliquer le procédé à des surfaces organiques plus complexes telles que des surfaces comprenant des polysaccharides, comme la cellulose pour le bois ou le papier, des fibres artificielles ou naturelles, comme le coton ou le feutre, ainsi que des polymères fluorés tels que le polytétrafluoroéthylène (PTFE) ou encore à des polymères porteurs de groupements basiques comme des amines tertiaire ou secondaire et par exemple les pyridines, comme les poly-4 et poly-2-vinylpyridines (P4VP et P2VP) ou plus généralement des polymères porteurs de groupements aromatiques et aromatiques nitrogénés.

Avantageusement, les première et seconde surfaces mises en oeuvre dans le cadre de la présente invention sont constituées d'un matériau identique ou différent, choisi dans le groupe constitué par les métaux, les alliages métalliques, le bois, le papier, le coton, le feutre de carbone, le silicium, les nanotubes, tels que les CNT, les matériaux graphitiques, comme le charbon, le graphène, les fullerènes et l'HOPG, les matériaux organiques tels que les polymères organiques, les polymères fluorés ou non fluorés et le diamant.

La nature de la première surface, i.e. la surface réactive, influe peu sur le procédé de l'invention. En effet, la présence d'une entité radicalaire et/ou ionique, d'un primaire d'adhésion ou d'un précurseur d'un primaire d'adhésion dans les différentes formes de mise en oeuvre décrites ci-après isole généralement le matériau constituant cette surface du reste du système.

Il est préférable que la seconde surface, i.e. la surface de revêtement, présente au moins un atome pouvant être impliqué dans une réaction chimique radicalaire et/ou ionique. Avantageusement, le matériau composant la surface de revêtement sera choisi parmi les matériaux susceptibles de réagir chimiquement avec des radicaux phényles ou des cations phényles. On peut notamment citer les matériaux graphitiques comme l'HOPG, le graphène, les CNT et les fullerènes ; les matériaux organiques, comme les polymères organiques, tels que la poly-4-vinyl pyridine (P4VP), ou l'acide polyacrylique ; et les métaux et alliages métalliques ; ceux-ci pouvant être sous la forme de particules ou agrégats métalliques.

Les zones de la première surface et de la seconde surface impliquées dans le procédé d'assemblage selon l'invention peuvent être de taille et/ou de forme identique ou différente. La forme de ces zones peut être simple ou complexe. Ces zones peuvent occuper de 0,01 % à 100 % de la surface totale de la première ou de la seconde surface. Généralement, la zone de la première surface (surface réactive) impliquée dans l'assemblage est plus grande que la zone de la seconde surface (surface de revêtement) impliquée dans l'assemblage.

Le terme « primaire d'adhésion » correspond, dans le cadre de la présente invention, à toute molécule organique susceptible, sous certaines conditions, de former soit des radicaux, soit des ions, et particulièrement des cations, et ainsi de participer à des réactions chimiques. De telles réactions chimiques pourront notamment être une chimisorption et, en particulier, un greffage chimique.

Le terme « greffage chimique » se réfère notamment à l'utilisation d'entités moléculaires extrêmement réactives (radicalaires ou ioniques et notamment cationiques) capables de former des liaisons de type liaison covalente avec une surface d'intérêt, lesdites entités moléculaires étant générées indépendamment de la surface sur laquelle elles sont destinées à être greffées. Ainsi, la réaction de greffage conduit à la formation de liaisons covalentes entre la zone de la surface de revêtement concernée et le dérivé du primaire d'adhésion.

Par « dérivé du primaire d'adhésion », on entend, dans le cadre de la présente invention, une unité chimique résultant du primaire d'adhésion, après que ce dernier a réagi avec une molécule d'intérêt ou par greffage chimique avec la surface de revêtement.

Le primaire d'adhésion est avantageusement un sel d'aryle clivable choisi dans le groupe constitué par les sels d'aryle diazonium, les sels d'aryle d'ammonium, les sels d'aryle phosphonium, les sels d'aryle iodonium et les sels d'aryle sulfonium. Dans ces sels, le groupe aryle est un groupe aryle qui peut être représenté par R tel que défini ci-après.

Dans une première variante de l'invention, le primaire d'adhésion est lié de façon directe à la première surface. Dans cette variante, le primaire d'adhésion est avantageusement lié à la zone de la première surface impliquée dans le procédé d'assemblage au moyen d'une liaison covalente. Ainsi, cette liaison covalente lie un atome de la zone de la première surface impliquée dans le procédé d'assemblage à un atome du primaire d'adhésion.

Dans une seconde variante de l'invention, le primaire d'adhésion est lié de façon indirecte à la zone de la première surface impliquée dans le procédé d'assemblage. Dans cette variante, le primaire d'adhésion et la zone de la première surface impliquée dans le procédé d'assemblage sont, chacun, liés à un agent de liaison qui maintient la liaison entre ledit primaire et ladite zone. Cet agent de liaison peut se présenter sous forme d'une entité unique dont une partie est liée au primaire d'adhésion et une autre partie à la zone de la première surface impliquée dans le procédé d'assemblage. Avantageusement, les différentes liaisons impliquées entre le primaire d'adhésion et l'agent de liaison, d'une part, et l'agent de liaison et la zone de la première surface impliquée dans le procédé d'assemblage sont des liaisons covalentes.

De façon alternative, l'agent de liaison comprend au moins deux entités, identiques ou différentes, liées entre elles, l'une étant liée au primaire d'adhésion et l'autre à la zone de la première surface impliquée dans le procédé d'assemblage. L'agent de liaison peut présenter plus de deux entités, identiques ou différentes, liées les unes aux autres, la première de ces entités étant liée au primaire d'adhésion et la dernière à la zone de la première surface impliquée dans le procédé d'assemblage. Avantageusement, les différentes liaisons impliquées entre le primaire d'adhésion et une des entités de l'agent de liaison, entre les différentes entités de l'agent de liaison et entre une des entités de l'agent de liaison et la zone de la première surface impliquée dans le procédé d'assemblage sont des liaisons covalentes. A titre d'exemple, l'agent de liaison peut se présenter sous forme d'un polymère ou copolymère, issu de plusieurs unités monomériques d'espèces chimiques identiques ou différentes.

Dans cette variante, l'agent de liaison peut se présenter sous forme d'un apprêt. Au sens de la présente invention, on entend par « apprêt », tout film de nature organique, notamment issu de plusieurs unités d'espèces chimiques organiques, lié préférentiellement de manière covalente à la zone de la première surface impliquée dans le procédé d'assemblage selon l'invention. Il s'agit particulièrement de films liés de manière covalente à cette zone et comprenant au moins une couche d'unités structurales de nature similaires. Selon l'épaisseur du film, sa cohésion est assurée par les liaisons covalentes qui se développent entre les différentes unités.

Cette variante permet notamment d'obtenir une localisation bien définie de la zone de la première surface impliquée dans le procédé selon l'invention.

En effet, dans le cadre d'une surface conductrice ou semi-conductrice, l'apprêt peut être préparé sur une zone sélectionnée de cette surface selon les étapes suivantes :
i) positionnement d'une microélectrode ME (i.e. une électrode dont au moins une des dimensions caractéristiques (le diamètre pour un disque) est au maximum de l'ordre de quelques dizaines de micromètres) à proximité de la surface de la zone sélectionnée ;
ii) mise en contact d'une solution liquide comprenant au moins un primaire d'adhésion tel que défini dans la présente invention et au moins un monomère polymérisable par voie radicalaire, identique ou différent dudit primaire d'adhésion, avec au moins ladite zone sélectionnée ;
iii) polarisation de ladite microélectrode et de la surface dudit substrat, le potentiel électrique de la surface étant plus cathodique que le potentiel de réduction du primaire d'adhésion organique mis en oeuvre à l'étape (ii).

En général, la séquence d'étapes est soit (i), (ii) et (iii), soit (i), (iii) et (ii).

La distance de travail (i.e. la distance entre la ME et la surface du support) choisie pour effectuer le procédé est celle pour laquelle le rapport entre la valeur de l'intensité du courant mesurée à la distance de travail et celle du courant mesurée à l'infini, i.e. loin de la surface, est compris entre 1,2 et 2,5. En général, une telle valeur correspond à une distance telle que le rapport entre la distance de travail et le rayon de la ME est compris entre 0,2 et 2.

Les monomères polymérisables par voie radicalaire mis en oeuvre dans le cadre du procédé de préparation de l'apprêt correspondent aux monomères susceptibles de polymériser en condition radicalaire après amorçage par une entité chimique radicalaire. Typiquement, il s'agit de molécules comportant au moins une liaison de type éthylénique. Les monomères vinyliques, notamment les monomères décrits dans la demande de brevet FR 05 02516 ainsi que dans le brevet FR 03 11491, sont particulièrement concernés.

Les monomères polymérisables par voie radicalaire sont avantageusement choisis dans le groupe constitué par l'acide acrylique, l'acétate de vinyle, l'acrylonitrile, le méthacrylonitrile, le méthacrylate de méthyle, le méthacrylate d'éthyle, le méthacrylate de butyle, le méthacrylate de propyle, le méthacrylate d'hydroxyéthyle, le méthacrylate d'hydroxypropyle, le méthacrylate de glycidyle et leurs dérivés ; les acrylamides et notamment les méthacrylamides d'amino-éthyle, propyle, butyle, pentyle et hexyle, les cyanoacrylates, les di-acrylates et di-méthacrylates, les tri-acrylates et tri-méthacrylates, les tétra-acrylates et tétra-méthacrylates (tels que le pentaérythritol tétra-méthacrylate), le styrène et ses dérivés, le parachloro-styrène, le pentafluoro-styrène, la N-vinyl pyrrolidone, la 4-vinyl pyridine, la 2-vinyl pyridine, les halogénures de vinyle, d'acryloyle ou de méthacryloyle, le di-vinylbenzène (DVB), et plus généralement les agents réticulants vinyliques ou à base d'acrylate, de méthacrylate, et de leurs dérivés.

La solution liquide comprenant au moins un primaire d'adhésion et au moins un monomère polymérisable par voie radicalaire peut en outre contenir un solvant protique avantageusement choisi dans le groupe constitué par l'eau, l'acide acétique, les solvants hydroxylés comme le méthanol et l'éthanol, les glycols liquides de faible poids moléculaire tels que l'éthylèneglycol, et leurs mélanges.

La solution liquide comprenant un primaire d'adhésion et un monomère polymérisable par voie radicalaire peut en outre contenir au moins un électrolyte support notamment choisi parmi les sels d'ammoniums quaternaires tels que les perchlorates, les tosylates, les tétrafluoroborates, les hexafluorophosphates, les halogénures d'ammoniums quaternaires à chaîne courte, le nitrate de sodium et le chlorure de sodium.

La solution liquide, comprenant un primaire d'adhésion et un monomère polymérisable par voie radicalaire peut en outre contenir au moins un tensioactif et ce, notamment pour améliorer la solubilité du monomère polymérisable par voie radicalaire. Une description précise des tensioactifs utilisables dans le cadre de l'invention est donnée dans la demande de brevet FR 2 897 876 à laquelle l'homme du métier pourra se référer. Un seul tensioactif ou un mélange de plusieurs tensioactifs peut être utilisé.

Ainsi, que la liaison du primaire d'adhésion à la zone de la première surface impliquée dans le procédé d'assemblage soit directe ou indirecte, le primaire d'adhésion est avantageusement lié de manière covalente à ladite zone de la première surface.

Les sels d'aryle clivables portés par la surface réactive (i.e. les sels d'aryle clivables liés de façon covalente à la zone de la première surface impliquée dans le procédé d'assemblage) sont également appelés, dans la présente, « sels d'aryle clivables supportés ». Toute utilisation du terme « supporté » dans la présente répond à la définition ci-dessus.

Parmi les sels d'aryle clivables supportés, on peut en particulier citer les composés de formule (I) suivante :

(première surface) - (B)ₙ-R-N₂⁺, A⁻ (I)

dans laquelle :
- (B)ₙ représente un agent de liaison,
- n est égal à 0 ou 1,
- A représente un anion monovalent et
- R représente un groupe aryle.

Comme précédemment défini, B peut représenter une entité unique, au moins deux entités identiques ou différentes ou même un apprêt tel(les) que décrit(es) ci-dessus.

A titre de groupes aryle R susceptibles d'être mis en oeuvre dans le cadre de la présente invention, notamment pour les sels d'aryle clivables supportés et, plus particulièrement, pour les composés de formule (I) ci-dessus, on peut avantageusement citer les structures carbonées aromatiques ou hétéroaromatiques, éventuellement mono- ou polysubstituées, constituées d'un ou plusieurs cycles aromatiques ou hétéroaromatiques comportant chacun de 3 à 8 atomes, le ou les hétéroatomes pouvant être N, O, P ou S. Le ou les substituants peuvent contenir un ou plusieurs hétéroatomes, tels que N, O, F, Cl, P, Si, Br ou S ainsi que des groupes alkyles en Cl à C6 notamment.

Au sein des primaires d'adhésion mis en oeuvre dans le cadre de la présente invention, notamment des sels d'aryle clivables supportés et, plus particulièrement des composés de formule (I) ci-dessus, le groupe aryle R est avantageusement choisi parmi les groupes aryles substitués par des groupements attracteurs d'électrons tels que NO₂, COH, les cétones, CN, CO₂H, NH₂ (sous forme de NH₃⁺), les esters et les halogènes. Les groupes aryle R particulièrement préférés sont les radicaux nitrophényle et phényle.

Au sein des composés de formule (I) ci-dessus, A peut notamment être choisi parmi les anions inorganiques tels que les halogénures comme I⁻, Br⁻ et Cl⁻, les halogénoborates tels que le tétrafluoroborate, les perchlorates et les sulfonates et les anions organiques tels que les alcoolates et les carboxylates.

A titre de primaire d'adhésion susceptible d'être mis en oeuvre dans le cadre de la présente invention, il est particulièrement avantageux d'utiliser un primaire choisi dans le groupe constitué par le tétrafluoroborate de phényldiazonium supporté, le tétrafluoroborate de 4-nitrophényldiazonium supporté, le tétrafluoroborate de 4-bromophényldiazonium supporté, le chlorure de 4-aminophényldiazonium supporté, le chlorure de 2-méthyl-4-chlorophényldiazonium supporté, le tétrafluoroborate de 4-benzoylbenzènediazonium supporté, le tétrafluoroborate de 4-cyanophényldiazonium supporté, le tétrafluoroborate du 4-carboxyphényldiazonium supporté, le tétrafluoroborate de 4-acétamidophényldiazonium supporté, le tétrafluoroborate de l'acide 4-phénylacétique diazonium supporté, le sulfate de 2-méthyl-4-[(2-méthylphényl)diazényl]benzènediazonium supporté, le chlorure de 9,10-dioxo-9,10-dihydro-1-anthracènediazonium supporté, le tétrafluoroborate de 4-nitronaphtalènediazonium supporté et le tétrafluoroborate de naphtalènediazonium supporté.En variante, le contre-ion peut être un chlorure.

Il est clair que la zone de la première surface mise en oeuvre dans le cadre du procédé d'assemblage selon la présente invention peut présenter plusieurs primaires d'adhésion et notamment plusieurs sels d'aryle clivables tels que précédemment définis, de nature identique ou différente.

Par « conditions non-électrochimiques », on entend dans le cadre de la présente invention en absence de tension électrique externe. Ainsi, les conditions non-électrochimiques mises en oeuvre dans le procédé selon l'invention et notamment à l'étape (b) de ce dernier sont des conditions qui permettent la formation d'entités radicalaires et/ou ioniques à partir du primaire d'adhésion, en l'absence de l'application d'une quelconque tension électrique à la surface réactive. Ces conditions impliquent des paramètres tels que, par exemple, la température, la nature du solvant, la présence d'un additif particulier, l'agitation, la pression alors que le courant électrique n'intervient pas lors de la formation des entités radicalaires. Les conditions non-électrochimiques permettant la formation d'entités radicalaires sont nombreuses et ce type de réaction est connu et étudié en détail dans l'art antérieur (Rempp & Merrill, Polymer Synthesis, 1991, 65-86, Hüthig & Wepf).

Il est ainsi par exemple possible d'agir sur l'environnement thermique, cinétique, chimique, photochimique ou radiochimique du primaire d'adhésion afin de le déstabiliser pour qu'il forme une entité radicalaire et/ou ionique. Il est bien entendu possible d'agir simultanément sur plusieurs de ces paramètres.

Dans le cadre de la présente invention, les conditions non-électrochimiques permettant la formation d'entités radicalaires et/ou ioniques sont typiquement choisies dans le groupe constitué par les conditions thermiques, cinétiques, chimiques, photochimiques, radiochimiques et leurs combinaisons. Avantageusement, les conditions non-électrochimiques sont choisies dans le groupe constitué par les conditions thermiques, chimiques, photochimiques, radiochimiques et leurs combinaisons entre elles et/ou avec les conditions cinétiques. Les conditions non-électrochimiques mises en oeuvre dans le cadre de la présente invention sont plus particulièrement des conditions chimiques.

L'environnement thermique est fonction de la température. Son contrôle est aisé avec les moyens de chauffage habituellement employés par l'homme du métier. L'utilisation d'un environnement thermostaté présente un intérêt particulier puisqu'il permet un contrôle précis des conditions de réaction.

L'environnement cinétique correspond essentiellement à l'agitation du système et aux forces de frottement. Il ne s'agit pas ici de l'agitation des molécules en elle-même (élongation de liaisons etc.), mais du mouvement global des molécules. L'application d'une pression permet notamment d'apporter de l'énergie au système pour que le primaire d'adhésion soit déstabilisé et puisse former des espèces réactives, radicalaires et/ou ioniques.

Enfin, l'action de rayonnements divers tels que rayonnements électromagnétiques, rayonnements γ, rayons UV, faisceaux d'électrons ou d'ions peut également déstabiliser suffisamment le primaire d'adhésion pour qu'il forme des radicaux et/ou des ions. La longueur d'onde employée sera choisie en fonction du primaire utilisé. Par exemple, une longueur d'onde d'environ 306 nm sera utilisée pour le 4-hexylbenzènediazonium supporté.

Dans le cadre des conditions chimiques, on emploie dans le milieu réactionnel un ou plusieurs amorceur(s) chimique(s). La présence d'amorceurs chimiques est souvent couplée à des conditions environnementales non chimiques, telles qu'exposées ci-dessus. Typiquement, un amorceur chimique dont la stabilité est moins grande que celle du primaire d'adhésion dans les conditions environnementales choisies va évoluer sous une forme instable qui agira sur le primaire d'adhésion et engendrera la formation d'entités radicalaires et/ou ioniques à partir de ce dernier. Il est également possible d'employer des amorceurs chimiques dont l'action n'est pas liée essentiellement aux conditions environnementales et qui peuvent agir sur de vastes plages de conditions thermiques ou encore cinétiques. L'amorceur sera de préférence adapté à l'environnement de la réaction, par exemple au solvant si un solvant est employé.

Il existe de nombreux amorceurs chimiques. On en distingue généralement trois types en fonction des conditions environnementales employées :
- les amorceurs thermiques dont les plus courants sont les peroxydes ou les composés azoïques. Sous l'action de la chaleur, ces composés se dissocient en radicaux libres. Dans ce cas, la réaction est effectuée à une température minimum correspondant à celle nécessaire à la formation de radicaux à partir de l'amorceur. Ce type d'amorceurs chimiques est en général utilisé spécifiquement dans un certain intervalle de températures, en fonction de leur cinétique de décomposition ;

- les amorceurs photochimiques ou radiochimiques qui sont excités par le rayonnement déclenché par irradiation (le plus souvent par UV, mais aussi par radiations γ ou par faisceaux d'électrons) permettent la production de radicaux par des mécanismes plus ou moins complexes. Le Bu₃SnH et l'I₂ appartiennent aux amorceurs photochimiques ou radiochimiques ;
- les amorceurs essentiellement chimiques, ce type d'amorceurs agissant rapidement et dans des conditions normales de température et de pression sur le primaire d'adhésion pour lui permettre de former des radicaux et/ou des ions. De tels amorceurs ont généralement un potentiel d'oxydoréduction qui est inférieur au potentiel de réduction du primaire d'adhésion utilisé dans les conditions de réaction. Selon la nature du primaire, il peut ainsi s'agir par exemple d'un métal réducteur, tel que du fer, zinc, nickel ; d'un métallocène ; d'un réducteur organique comme l'acide hypophosphoreux (H₃PO₂) ou l'acide ascorbique ; d'une base organique ou inorganique dans des proportions suffisantes pour permettre une déstabilisation du primaire d'adhésion. Avantageusement, le métal réducteur utilisé en tant qu'amorceur chimique se présente sous forme finement divisée, comme de la laine (également appelée plus communément « paille ») métallique ou de la limaille métallique. Généralement, lorsqu'une base organique ou inorganique est utilisée comme amorceur chimique, un pH supérieur ou égal à 4 est généralement suffisant. Des structures de type réservoir de radicaux, comme des matrices polymériques préalablement irradiées par un faisceau d'électrons ou par un faisceau d'ions lourds et/ou par l'ensemble des moyens d'irradiations cités précédemment, peuvent également être employées en tant qu'amorceurs chimiques pour déstabiliser le primaire d'adhésion et conduire à la formation d'entités radicalaires et/ou ioniques à partir de ce dernier.

Selon les conditions employées lors de l'étape (b) du procédé selon l'invention, il est bien entendu possible d'utiliser un solvant. Ainsi, par exemple, lorsque des conditions chimiques sont employées et qu'un amorceur est utilisé, celui-ci sera avantageusement placé dans une solution au contact de la surface réactive pour permettre la déstabilisation du primaire d'adhésion et la formation des espèces réactives. Avantageusement, le solvant sera choisi de telle sorte qu'il ne réagisse pas de manière significative avec la surface réactive. Ainsi, par exemple, si le primaire d'adhésion est un sel de diazonium, il est recommandé d'employer un solvant non-protique.

Il est utile de se reporter à [Chem. Mater. 2007, 19,6323-6330] pour la formation d'espèces actives.

La mise en contact de la première surface (surface réactive) avec la seconde surface (surface de revêtement) ou une molécule d'intérêt peut être effectuée, à l'étape (c) du procédé de l'invention, selon différentes dispositions.

Selon une première forme de mise en oeuvre, la mise en contact de l'étape (c) du procédé selon l'invention est effectuée de façon directe. Ainsi la surface réactive est placée directement au contact de la surface de revêtement ou de la molécule d'intérêt. Par exemple, une surface de revêtement composée de graphène ou encore d'une membrane polymère peut être directement placée sur la surface réactive. De la même manière, la molécule d'intérêt peut être déposée directement sur la surface.

Selon ce mode de réalisation, il est possible d'appliquer une pression à la surface de revêtement au contact de la surface réactive pour déstabiliser le primaire. Il est également possible de chauffer le système, par exemple vers 100°C, ou de l'exposer à une illumination, comme une illumination UV dans le cas des diazonium, particulièrement dans le cas où l'assemblage implique des molécules d'intérêt. L'exemple II.1.1 ci-après correspond à cette première forme de mise en oeuvre.

Selon une seconde forme de mise en oeuvre, la mise en contact de l'étape (c) du procédé selon l'invention est effectuée en solution. Généralement, la surface de revêtement ou la molécule d'intérêt est placée en solution puis la solution est directement mise au contact de la surface réactive. Le solvant de la solution peut ensuite être évaporé pour placer ainsi la surface de revêtement ou la molécule directement au contact de la surface réactive. Cette forme de mise en oeuvre permet une grande variabilité de méthode d'enduction (tournette, trempage, spray, pinceau, ...). Les exemples II.2 ci-après correspondent à cette forme de mise en oeuvre.

Tout solvant connu de l'homme du métier est susceptible d'être utilisé dans cette forme de mise en oeuvre. L'homme du métier saura en fonction de la molécule d'intérêt, de la surface de revêtement, des entités radicalaires et/ou ioniques, de la surface réactive impliquées, quel solvant utiliser. A titre d'exemples de solvants utilisables, on peut citer le diméthyl formamide et la n-méthyl pyrrolidone.

Concernant l'immobilisation de molécules biologiques sur la zone de la première surface, cette dernière est avantageusement réalisée en milieu organique, en milieu aqueux ou en milieu tampon qui peut être, de façon non limitative, du tampon TRIS (pour « tris(hydroxyméthyl)aminométhane »), du tampon phosphate, du tampon acétate... L'utilisation de milieu organique est généralement privilégiée pour l'immobilisation de produits synthétiques biologiquement actifs tels que, à titre non limitatif, des peptides synthétiques semi-protégés généralement de nature hydrophobe en raison de la protection des groupes hydrophiles, des peptides synthétiques déprotégés hydrophobes en raison d'une grande proportion d'acides aminés de type alkyles ou hydrophobes au sein de la séquence primaire dudit peptide ou de molécules biologiques hydrophobes telles que, à titre non limitatif, des fibres de collagène, des protéines transmembranaires, la chitine et ses dérivés. Typiquement, les solvants organiques utilisés lors de l'étape (c) impliquant, comme molécules d'intérêt, des molécules biologiques ou biologiquement actives, sont choisis dans le groupe constitué par l'acétonitrile, la diméthylformamide, le diméthylsulfoxide (DMSO). Le DMSO sera privilégié en raison de sa faible toxicité et de son utilisation autorisée et courante en biologie. Cependant, l'utilisation de milieux aqueux ou de solutions tampons sera privilégiée si la molécule à immobiliser présente une solubilité dans ces milieux même si cette solubilité est faible.

Dans une première variante du procédé de l'invention, les étapes (b) et (c) du procédé sont réalisées de façon simultanée. Dans cette variante, l'étape correspondant aux étapes (b) et (c) réalisées de façon simultanée consiste à mettre concomitamment ladite zone de ladite première surface présentant au moins un primaire d'adhésion à des conditions non électrochimiques pour obtenir, sur ladite zone, au moins une entité radicalaire et/ou ionique et dans des conditions de contact avec la seconde surface ou avec ladite molécule d'intérêt. La réaction peut alors être spontanée ou activée.

Dans cette variante, la seconde surface ou la molécule d'intérêt peut directement réagir avec le primaire d'adhésion présent sur la première surface, la seconde surface et la molécule d'intérêt jouant le rôle d'amorceur chimique tel que précédemment défini, i.e. un amorceur chimique qui active le primaire d'adhésion pour donner une forme radicalaire et/ou ionique et qui éventuellement réagit avec cette dernière. Avantageusement, dans cette variante, la surface de revêtement ou la molécule d'intérêt est placée en solution comme précédemment défini.

Dans une seconde variante du procédé de l'invention, les étapes (b) et (c) du procédé ne sont pas réalisées de façon concomitante. Dans cette variante, il est avantageux que, si une solution réactionnelle est utilisée, cette dernière soit dégazée.

Selon un mode de réalisation particulier, le procédé comporte en outre une étape de préparation de la surface réactive présentant un primaire d'adhésion mise en oeuvre à l'étape (b) du procédé selon l'invention. Cette étape supplémentaire correspond à l'étape (a) du procédé selon l'invention. La surface réactive présentant un primaire d'adhésion est généralement préparée à partir d'une surface présentant un précurseur de primaire d'adhésion. Cette dernière peut notamment être réalisée par enduction de la première surface et, plus particulièrement, sur la zone de la première surface impliquée dans l'assemblage pour former sur celle-ci un apprêt comportant un précurseur de primaire d'adhésion.

Dans le cadre de l'invention, par « précurseur de primaire d'adhésion », il faut comprendre une molécule séparée du primaire d'adhésion par une étape opératoire unique et aisée à mettre en oeuvre. Généralement les précurseurs présentent une stabilité plus importante que les primaires d'adhésion dans les mêmes conditions environnementales. L'homme du métier connaît différents couples « précurseur de primaire d'adhésion »/« primaire d'adhésion ». Ainsi, par exemple, les arylamines sont des précurseurs des sels d'aryle diazonium. En effet, par simple réaction d'oxydation, par exemple, avec NaNO₂ dans un milieu aqueux acide, ou avec NOBF₄ en milieu organique, il est possible de former les sels d'aryle diazonium correspondant. Dans ces conditions, le passage d'un apprêt comportant un précurseur à un apprêt comportant le primaire correspondant est facile. La définition du terme « apprêt » telle que précédemment donnée pour l'agent de liaison s'applique également au précurseur de primaire d'adhésion *mutatis mutandis.* Ainsi, ladite zone de ladite première surface présentant au moins un précurseur d'un primaire d'adhésion est une zone de ladite première surface à laquelle est lié, préférentiellement de façon covalente, un film organique présentant au moins un précurseur d'un primaire d'adhésion.

A titre de précurseur de primaire d'adhésion, on peut notamment citer les précurseurs de sels d'aryle diazonium supportés que sont les amines supportées de formule (II) suivante :

(première surface)-(B)ₙ-R-NH₂ (II)

dans laquelle B, R et n sont tels que définis pour la formule (I).

A titre de précurseur de primaire d'adhésion susceptible d'être mis en oeuvre dans le cadre de la présente invention, il est particulièrement avantageux d'utiliser un précurseur choisi dans le groupe constitué par la phénylamine supportée, la 4-nitrophénylamine supportée, la 4-bromophénylamine supportée, la 4-aminophénylamine supportée, la 2-méthyl-4-chlorophénylamine supportée, la 4-benzoylbenzèneamine supportée, la 4-cyanophénylamine supportée, la 4-carboxyphénylamine supportée, la 4-acétamidophénylamine supportée, l'acide 4-amino benzoïque supporté, la 2-méthyl-4-[(2-méthylphényl)diazényl]amine supportée, la 9,10-dioxo-9,10-dihydro-1-anthracèneamine supportée, la 4-nitronaphtalèneamine supportée et la naphtalèneamine supportée.

Différents procédés d'enduction sont connus de l'art antérieur et peuvent être employés selon la nature de la première surface pour obtenir une surface dont au moins une zone présente au moins un précurseur de primaire d'adhésion susceptible d'être utilisée à l'étape (a) du procédé selon l'invention. Ces procédés peuvent être des procédés chimiques ou électrochimiques. Il peut, par exemple, s'agir de trempage, de dépôt à la tournette, au pinceau, ou par projection durant la phase de dépôt, ou par pression et contact direct si l'enduit est solide. Typiquement, lorsque l'utilisateur désire que l'apprêt soit lié, de manière covalente à la surface de base, l'enduction pourra être effectuée par un procédé de greffage de film organique. Quel que soit le type de surface mise en oeuvre, le greffage utilisé pourra être un greffage chimique tel que décrit dans [Chem. Mater. 2007, 19,6323-6330]. Lorsque la surface de base est conductrice ou semi-conductrice, ce greffage peut avantageusement consister en un électrogreffage tel que décrit dans [Chem. Mater. 2006, 18, 4755-4763] ou un électrogreffage impliquant une microélectrode tel que précédemment décrit. Le procédé d'enduction conduit généralement à la formation d'un apprêt comportant un précurseur de primaire d'adhésion. Ainsi, par exemple, par greffage chimique à partir d'arylediamines, il est possible de former un apprêt, ici un film organique, dont la surface comporte des précurseurs de primaire d'adhésion sous la forme d'arylamines supportées.

L'homme du métier saura déterminer les conditions adéquates à utiliser, lors de l'étape (a) du procédé, en fonction du type de précurseur mis en oeuvre et du primaire d'adhésion à obtenir. Ainsi, la transformation des fonctions amine en sels d'aryle diazonium peut être réalisée en une seule étape à l'aide de NaNO₂ par simple réaction d'oxydation, dans un milieu aqueux acide, ou à l'aide de NOBF₄, en milieu organique.

L'utilisation d'un apprêt permet notamment de choisir précisément le positionnement du primaire d'adhésion sur la surface. Dans ces conditions, un contrôle total de la localisation des zones de surface mises en oeuvre dans l'assemblage peut être obtenu. En effet, l'enduction n'est réalisée que dans les zones souhaitées par l'utilisateur.

L'utilisation d'un apprêt permet en outre d'augmenter la planéité des surfaces. En effet, lorsque la surface de base est rugueuse, il est possible de la lisser par enduction d'une couche d'apprêt suffisamment importante pour augmenter la planéité de la surface obtenue. L'enduction permet d'assurer au besoin le comblement des aspérités de la surface de base. L'invention permet ainsi d'adapter la morphologie de la surface réactive pour augmenter le contact possible avec la surface de revêtement.

L'épaisseur de l'apprêt peut varier de la monocouche moléculaire à une épaisseur de plusieurs nanomètres jusqu'à l'échelle du micron. Il est ainsi possible de moduler la conductivité électrique de l'apprêt car, à partir de quelques nanomètres, les films organiques deviennent isolants.

Les primaires d'adhésion étant des molécules particulièrement réactives, il est possible de conserver la surface réactive avec plus de facilité lorsqu'elle comporte un précurseur de primaire d'adhésion plutôt qu'un primaire d'adhésion en tant que tel.

Les primaires d'adhésion, comme les sels de diazonium, sont le verrou chimique du procédé qui permet d'immobiliser des entités comme les graphènes sur des surfaces. L'invention permet de réaliser une chimie difficile à faire avec des matériaux de très faible réactivité chimique tels que les graphènes (réseau plan graphitique thermodynamiquement très stable). Des matériaux graphitiques tels que les CNT ou les fullerènes, qui présentent des courbures de réseau importantes, peuvent être également employés. Au-delà encore, les primaires employés, comme les sels de diazonium, sont susceptibles de réagir avec un grand nombre de composés chimiques nucléophiles (bases organiques COO⁻, SO₃²⁻, NH₂, Pyridines, etc ...) .

L'invention se différencie des procédés de l'art antérieur notamment en ce qu'à la différence d'un collage classique pour lequel les fonctions chimiques responsables de l'adhésion sont amenées avec l'adhésif, les fonctions chimiques responsables de l'adhésion sont déjà présentes sur la surface, ici la surface réactive, qui peut être qualifiée de pré-adhésive.

De plus, le procédé selon l'invention peut comprendre une étape additionnelle de structuration de la zone de la première surface mise en oeuvre. Cette structuration consiste à modifier la surface réactive et, plus particulièrement, à diminuer la taille de cette surface réactive et/ou à diminuer le nombre d'entités radicalaires et/ou ioniques sur cette surface.

Ainsi, la surface réactive peut être soumise à une irradiation UV sous une lampe UV (spectre 300-500 nm, 200 W) pendant quelques minutes. Typiquement pour une couche portant les sels de diazonium de 2 nm, un temps d'exposition aux UV de 3 à 10 min permet la destruction de tous les sels de diazonium présents à la surface. Ce résultat a pu être suivi par spectrométrie IR avec la disparition complète du pic à 2270 cm⁻¹. La couche ainsi obtenue est appelée « couche morte » et ne permet plus l'assemblage ou l'immobilisation d'une autre surface ou d'une molécule d'intérêt.

Le temps d'irradiation aux UV pour obtenir la « couche morte » est fonction de l'épaisseur de cette dernière. En variant le temps d'irradiation et/ou l'intensité de l'irradiation (puissance inférieure), la densité des entités radicalaires et/ou ioniques au niveau de la zone de la première surface peut être contrôlée permettant de moduler le nombre de sites actifs par unité de surface. Ce procédé permet de moduler la densité de molécules d'intérêt par unité de surface du support.

En variante, la structuration de la zone de la première surface mise en oeuvre dans le procédé de la présente invention peut être effectuée préalablement à la préparation de la surface réactive.

Cette variante peut aussi bien impliquer soit l'électrogreffage d'un apprêt utilisant une microélectrode tel que précédemment décrit, soit l'utilisation d'un tampon à appliquer sur le support.

Ce tampon qui peut être assimilé à un masque est appliqué préalablement aux étapes (a) et (b) du procédé selon l'invention. Il correspond typiquement à une entité physique qui n'est ni greffée à la surface, ni liée de manière covalente à celle-ci. Il peut notamment s'agir d'un matériau massif ou d'une mince couche de matière, typiquement de quelques Angstroms à quelques microns, généralement de nature organique, déposé(e) sur la surface.

Le tampon permet de « masquer » localement la réactivité chimique de la surface à l'égard des radicaux générés durant le procédé et entraîne ainsi la formation de manière contrôlée d'un film uniquement sur les parties de la surface exposées à la solution, les zones de la surface du support équipées par le masque étant préservées de la formation du film organique. La surface du support solide mise au contact de la solution liquide telle que précédemment définie comporte ainsi typiquement au moins une zone recouverte d'un masque. Après élimination du masque en fin d'opération, la surface qui était protégée, contrairement à celle qui n'était pas équipée d'un masque, ne comporte pas de film greffé.

De préférence, le masque sera constitué d'une mince couche de matière inorganique ou organique agissant comme une couche de moindre cohésion aisément éliminable dans des conditions douces. Une couche de matière est considérée comme telle dans la mesure où elle ne nécessite pas d'employer des conditions extrêmes nuisibles au film greffé pour être éliminée. Typiquement les conditions douces correspondent à un simple lavage chimique, effectué généralement à l'aide d'un solvant dans lequel le masque est soluble, à un traitement ultrasonore dans un solvant dans lequel le masque est soluble ou à une élévation de la température. Il est bien entendu souhaitable que le masque ne soit pas soluble dans le solvant présent dans la solution liquide *i.e*. employé dans le cadre de la réaction de greffage. Ainsi il est recommandé d'employer un masque qui présente une affinité pour la surface supérieure à celle qu'il présente pour le solvant de réaction.

La matière constituant le masque peut ainsi être choisie dans une large gamme. Elle sera généralement choisie en fonction de la nature du support solide.

Le masque peut réagir avec les radicaux ou ions générés au cours du procédé. Dans tous les cas, il est possible de l'éliminer pour découvrir les zones de la surface du support solide protégées du greffage sur lesquelles aucun film organique ne sera observé (assimilable aux méthodes dites de « lift-off » en lithographie).

Les techniques de dépôts de masque sont bien connues de l'homme du métier. Il peut s'agir notamment d'enduction, de vaporisation ou encore d'immersion. Ainsi, le masque, sous forme d'une mince couche de matière, peut-être par exemple déposé soit par dessin direct à partir d'un feutre (type crayon) imprégné de la matière choisie. Sur du verre, il est, par exemple, possible d'employer, à titre de masque, un marqueur tel que ceux proposés en papeterie ou encore des corps gras. Il est également possible d'employer le procédé dit « du tampon ». Cette technique est applicable notamment dans le cas de support solide présentant une surface complexante pour les atomes de soufre, comme une surface d'or, dans ce cas le masque sera généralement composé d'alkylthiols, en particulier d'alkylthiols à longue chaine, souvent en C15-C20 et typiquement en C18 (technique dite « de la microimpression » ou « microcontact printing» en anglais). Plus généralement, les techniques de lithographie classiques peuvent être employées pour former le masque : spin-coating, puis insolation à travers un masque physique ou via un faisceau de lumière ou de particules pilotable, puis révélation.

La présente invention trouve des applications particulièrement intéressantes dans le domaine de la biologie. En effet, le support mis en oeuvre dans le cadre de la présente invention peut se présenter sous diverses formes, de taille variable et utiles en biologie. A titre d'exemples et de façon non exhaustive, il peut se présenter sous forme de lames, de microplaques notamment de microplaques à 12, 24 ou 96 puits, de microplaquettes, de particules, de billes, de microbilles, de fibres, de feutres, de tubes tels que des tubes à hémolyse ou de microcanaux de type capillaires, des colonnes ou des microcolonnes telles que des colonnes SPIN™, de supports utilisés pour les biocapteurs ou les biopuces. Ces différents types de support peuvent avoir des tailles variant de quelques centaines de micromètres à plusieurs centimètres. Dans ces applications en biologie, les molécules d'intérêt à assembler ou à immobiliser, ci-après désignées par « molécules biologiques ou biologiquement actives » seront avantageusement choisies dans le groupe constitué par les peptides ; les protéines telles que la gélatine, la protéine A, la protéine G, la streptavidine, la biotine, une enzyme ; les anticorps et fragments d'anticorps ; les récepteurs cellulaires ou membranaires ; les polysaccharides comme les glycoaminoglycannes et notamment l'héparine ; des cellules ou parties cellulaires telles que des organites ou des membranes cellulaires et les acides nucléiques tels que ADN et ARN.

Ainsi, comme précédemment expliqué, la présente invention peut être utilisée pour la préparation de biopuces ou de biocapteurs. Cette préparation peut présenter différents modes de réalisation :
- une couche d'un précurseur d'un primaire d'adhésion du type diazonium est formée sur un support conducteur ou isolant, de nature organique ou inorganique, la couche est activée pour former des espèces radicalaires et/ou ioniques puis :
- la molécule biologique ou biologiquement active est déposée sur la surface totale du support,
- ou la molécule biologique ou biologiquement active est déposée grâce à un système de micro ou nano-fluidique de façon directe ou séquentielle sous la forme de goutte, une autre molécule pouvant ainsi être introduite grâce au dépôt d'une goutte et ainsi de suite,
- ou le support activé est trempé dans la solution contenant la molécule biologique ou la molécule biologiquement active.

Un exemple d'un précurseur d'un primaire d'adhésion du type diazonium est notamment un composé de structure polyphénylénique.

Il est possible, pour toutes les variantes précédemment définies, de réaliser une étape de structuration de la surface du support mis en oeuvre dans le procédé selon l'invention.

Cette structuration peut consister en une étape intermédiaire avec le dépôt d'un masque puis irradiation à l'UV et ce, avant le dépôt ou l'immobilisation de la molécule biologique ou la molécule biologiquement active. Ainsi, une grille ou un masque déposé(e) sur le support activé possédant une géométrie particulière permet l'obtention de plot (carré, cercle...) puis l'ensemble est soumis à l'irradiation UV pour un temps préalablement défini. Les zones du primaire d'adhésion irradiées à l'UV seront maintenant considérées comme inactives. Les zones du primaire d'adhésion non irradiées par l'UV seront toujours considérées comme actives. Elles seront préférentiellement' situées à la surface des plots que l'on veut créer.

Une variante de cette structuration peut consister, préalablement à la mise en oeuvre du procédé selon la présente invention, à l'utilisation d'un tampon servant à masquer certaines zones de la surface tel que précédemment défini.

Il est possible, pour toutes les variantes précédemment définies, d'obtenir la couche de précurseur d'un primaire d'adhésion du type diazonium :
- en formant une couche d'un tel précurseur de façon électrochimique sur un support conducteur ou de façon chimique sur un support conducteur ou isolant, et notamment tel que décrit dans la demande internationale WO 2008/078052, le support pouvant être de nature organique ou inorganique ;
- un dépôt sous forme de gouttes contenant la diamine activée de manière chimique sur un support conducteur ou isolant de nature organique ou inorganique, créant un patterning par un système de nano ou micro-fluidique et donc de plot de précurseur d'un primaire d'adhésion du type diazonium ;

- un dépôt sous forme de gouttes contenant la diamine activée de manière électrochimique avec un système nano ou micro-fluidique avec une contre-électrode sur un support conducteur de nature organique ou inorganique, créant un patterning par un système de nano ou micro-fluidique et donc de plot de précurseur d'un primaire d'adhésion du type diazonium ;
- la formation directe d'une couche de diazonium sur une puce (qui possède une structuration de surface en plot par exemple) par procédé électrochimique, chacun des plots métalliques de la puce étant recouvert d'une couche de diazonium. Chaque plot métallique est alors recouvert par une goutte d'un volume défini contenant la ou les molécule(s) biologique(s) d'intérêt.

La présente invention concerne également un support solide dont la surface présente au moins une zone telle que précédemment définie, à savoir avec au moins une entité radicalaire et/ou ionique, avec au moins un primaire d'adhésion, ou avec au moins un précurseur d'un primaire d'adhésion. Lesdites entités, primaire et précurseur, peuvent être liées de façon directe ou indirecte à ladite surface et ce dans les différentes formes de mise en oeuvre précédemment envisagées. Tout ce qui a été précédemment défini pour la surface (forme, nature, taille,...) s'applique *mutatis mutandis* au présent support solide.

La présente invention concerne aussi l'utilisation d'un support solide tel que précédemment défini pour préparer une biopuce ou un biocapteur et une biopuce ou un biocapteur comprenant un support solide dont la surface présente au moins une zone avec au moins une entité radicalaire et/ou ionique telle que précédemment définie ayant réagi avec un élément biologique choisi dans le groupe constitué par les peptides ; les protéines telles que la gélatine, la protéine A, la protéine G, la streptavidine, la biotine, une enzyme ; les anticorps et fragments d'anticorps ; les récepteurs cellulaires ou membranaires ; les polysaccharides comme les glycoaminoglycanes et notamment l'héparine ; des cellules ou parties cellulaires telles que des organites ou des membranes cellulaires et les acides nucléiques tels que ADN et ARN.

La présente invention concerne également un kit d'éléments susceptible d'être utilisé lors de la mise en oeuvre d'un procédé tel que précédemment défini. Un tel kit comprend notamment :
- dans un premier compartiment, un support solide dont la surface présente au moins une zone telle que précédemment définie, à savoir avec au moins une entité radicalaire et/ou ionique, avec au moins un primaire d'adhésion, ou avec au moins un précurseur d'un primaire d'adhésion.
- éventuellement, dans un second compartiment, au moins un élément nécessaire pour élaborer le primaire d'adhésion à partir de son précurseur (par exemple, une solution de NaNO₂ dans un milieu aqueux acide, ou une solution de NOBF₄, en milieu organique) et/ou au moins un élément nécessaire pour élaborer une espèce radicalaire et/ou ionique à partir du primaire d'adhésion tel qu'un amorceur chimique,
- éventuellement, dans un troisième compartiment, un autre support solide ou un nano-objet dont la surface correspond à la seconde surface telle que précédemment définie ou une molécule d'intérêt telle que précédemment définie à immobiliser.

La présente invention concerne également l'utilisation d'un support solide dont au moins une zone de la surface comprend au moins une entité radicalaire et/ou ionique pour immobiliser sur ce dernier un autre support solide ou un nano-objet dont la surface correspond à la seconde surface telle que précédemment définie ou une molécule d'intérêt telle que précédemment définie. Ainsi, la présente invention concerne l'utilisation d'un support solide dont au moins une zone de la surface comprend au moins une entité radicalaire et/ou ionique pour immobiliser sur ce dernier un nanotube de carbone, simple paroi ou multi parois, un feuillet de graphène ou un nanofilm de silicium.

Plus particulièrement, la présente invention concerne l'utilisation d'un procédé tel que précédemment décrit ou d'un support solide dont au moins une zone de la surface comprend au moins une entité radicalaire et/ou ionique telle que définie pour exfolier des feuillets de graphène.

De plus, la présente invention concerne l'utilisation d'un procédé tel que précédemment décrit ou d'un support solide dont au moins une zone de la surface comprend au moins une entité radicalaire et/ou ionique telle que définie pour métalliser la zone de ladite première surface. En effet, dans le cas où la seconde surface mise en oeuvre est un nano-objet (NB) et notamment une nanoparticule (NP), il est possible d'employer une solution comportant un ou plusieurs sels métalliques susceptibles d'être réduits par le NB immobilisé sur la première surface suite à la mise en oeuvre du procédé selon l'invention. En général, le support sur lequel est immobilisé le NB sera directement plongé dans une solution comportant un ou plusieurs sels métalliques susceptibles d'être réduits par le NB.

La présente invention trouve également une application dans le domaine du traitement des surfaces. En effet, elle peut être mise en oeuvre pour traiter, de façon durable, un matériau et notamment pour modifier les propriétés telles que l'énergie de surface également appelée « tension de surface », « tension superficielle », « énergie d'interface » ou « tension interfaciale » d'au moins une de ses surfaces et ainsi pour modifier la mouillabilité de cette surface. L'invention permet notamment de modifier les propriétés d'interface entre ledit matériau et un liquide.

Par « modifier l'énergie de surface », on entend dans le cadre de la présente invention aussi bien augmenter que diminuer l'énergie de surface notamment par rapport à un liquide donné qu'il soit hydrophile ou hydrophobe. Le procédé selon la présente invention permet de modifier (i.e. augmenter ou diminuer) l'angle de contact d'un liquide disposé sur la surface ainsi traitée par rapport à l'angle de contact du même liquide disposé sur ladite surface non traitée. Avantageusement, le procédé selon la présente invention est un procédé qui permet de modifier (i.e. augmenter ou diminuer) la mouillabilité de ladite surface.

Dans cette application, la surface dont on souhaite modifier l'énergie de surface est une « surface réactive » telle que précédemment définie. Elle peut être de toutes les natures précédemment envisagées (organique, inorganique, isolante, conductrice ou semi-conductrice). Plus particulièrement, cette surface est une surface en verre tel qu'un verre plat notamment utilisé dans le bâtiment, l'architecture, l'automobile, le vitrage et la miroiterie, un verre d'aquarium, un verre de lunetterie ou un verre optique. En outre, l'épaisseur du revêtement organique sur la surface réactive assemblée avec la molécule d'intérêt est facilement contrôlable, ce qui ne modifie pas les propriétés optiques du matériau.

Les molécules hydrophobes sont typiquement insolubles dans les solvants protiques et particulièrement dans l'eau. La solubilité de ces molécules est finie et elles peuvent former des phases non miscibles avec les solvant protiques tels que l'eau. Elles comprennent généralement au moins un groupe chimique qui est qualifié d'hydrophobe. Le groupement hydrophobe participe à la modification de l'énergie de surface. Le groupement hydrophobe est choisi, de façon avantageuse, dans le groupe constitué par
- un alkyle, linéaire, ramifié ou cyclique, en C3 à C50, notamment en C6 à C30 et, en particulier, en C10 à C20 pouvant comporter éventuellement au moins une insaturation (double ou triple liaison), au moins un hétéroatome et/ou au moins une substitution,
- un aryle en C3 à C50, notamment en C6 à C30 et, en particulier, en C10 à C20 pouvant comporter éventuellement au moins une substitution, cette substitution peut être un alkyle linéaire, branché ou cyclique en C3 à C50, notamment en C6 à C30 et, en particulier, en C10 à C20 qui peut éventuellement comprendre au moins une insaturation (double ou triple liaison) et/ou au moins un hétéroatome, et
- un (poly)cycle en C6 à C50, notamment en C6 à C30 et, en particulier, en C10 à C20 pouvant comporter éventuellement au moins une insaturation (double ou triple liaison), au moins un hétéroatome et/ou au moins une substitution.

Ladite substitution est avantageusement une substitution par un alkyle en C1 à C6 et/ou par un halogène et notamment un fluor.

Une molécule d'intérêt utilisable pour cette application est notamment un tensioactif et, en particulier, un tensioactif fluoré tel que ceux contenus dans les compositions commercialisés par DuPont sous la marque Zonyl.

La présente invention concerne donc un procédé pour modifier l'énergie de surface d'au moins une surface d'un solide consistant à assembler ladite surface avec une molécule hydrophobe telle que précédemment définie et ce, selon un procédé d'assemblage tel que précédemment défini.

Avantageusement, ce procédé de modification de l'énergie de surface comprend une étape supplémentaire, suite à cet assemblage, consistant à soumettre ladite surface assemblée avec ladite molécule à un traitement thermique. Ce dernier traitement permet, en effet, d'améliorer la modification de l'énergie de surface. Ledit traitement thermique consiste à soumettre ledit film greffé à une température comprise entre 60 et 180°C, notamment entre 90 et 150°C et, en particulier, de l'ordre de 120°C (i.e. 120°C ± 10°C) et ce, pendant une durée généralement comprise entre 1 h et 3 j, notamment entre 6 h et 2 j et, en particulier, entre 12 et 24 h. Cette étape de traitement thermique peut être mise en oeuvre dans une étuve ou dans un four.

D'autres caractéristiques et avantages de la présente invention apparaîtront encore à la lecture des exemples ci-après donnés à titre illustratif et non limitatif et faisant référence aux figures annexées.

### BRÈVE DESCRIPTION DES DESSINS

La Figure 1 est une représentation schématique de l'exfoliation du graphène.
La Figure 2 présente des exemples de réactions chimiques créant un liant covalent entre le primaire d'adhésion et le revêtement.
La Figure 3 présente des spectres IR par ATR du film précurseur de primaire d'adhésion déposé chimiquement, 40 min (type polyaminophénylène) avant (a) et après (b) un rinçage à l'eau suivi de 5 min sous ultrasons dans l'acétone.
La Figure 4 présente des spectres IR par ATR du film précurseur de primaire d'adhésion déposé électrochimiquement, 30 mCb à -0,6 V (type polyaminophenylène): avant (a) et après (b) un rinçage à l'eau suivi de 5 minutes sous ultrasons dans l'acétone.
La Figure 5 présente les spectres IR (a) d'une surface revêtue par une couche mince d'amines aromatiques, surface dite précurseur de primaire d'adhésion préparée chimiquement, (b) de la même surface devenue primaire d'adhésion après traitement NOBF₄ (10⁻² M) 30 sec dans CH₃CN.
La Figure 6 présente des spectres IR (a) d'une surface revêtue par une couche mince d'amine aromatique, surface dite précurseur de primaire d'adhésion préparée électrochimiquement, (b) de la même surface devenue primaire d'adhésion après traitement NOBF₄ (10⁻² M) 30 sec dans CH₃CN.
La Figure 7 présente la re-diazotisation en milieu aqueux (donc absence de la bande BF₄⁻) : (a) Spectre IR de la surface précurseur, (b) Spectre IR de la surface primaire.
La Figure 8 présente le greffage de multi-feuillets de graphène sur une surface diazotisée. Les Figures 8A, 8B, 8C et 8D représentent différentes zones à différents grossissements.
La Figure 9 présente le greffage de CNT sur surface diazotisée. Les Figures 9A, 9B, 9C et 9D correspondent à différents grossissements de la surface greffée, respectivement 80000x, 20000x, 50000x et 35000x.
La Figure 10 présente le spectre IR d'une surface revêtue d'un précurseur de primaire de type polyaminophénylène (a), d'un primaire (b) et après réaction avec l'acétyle pyridine (c).

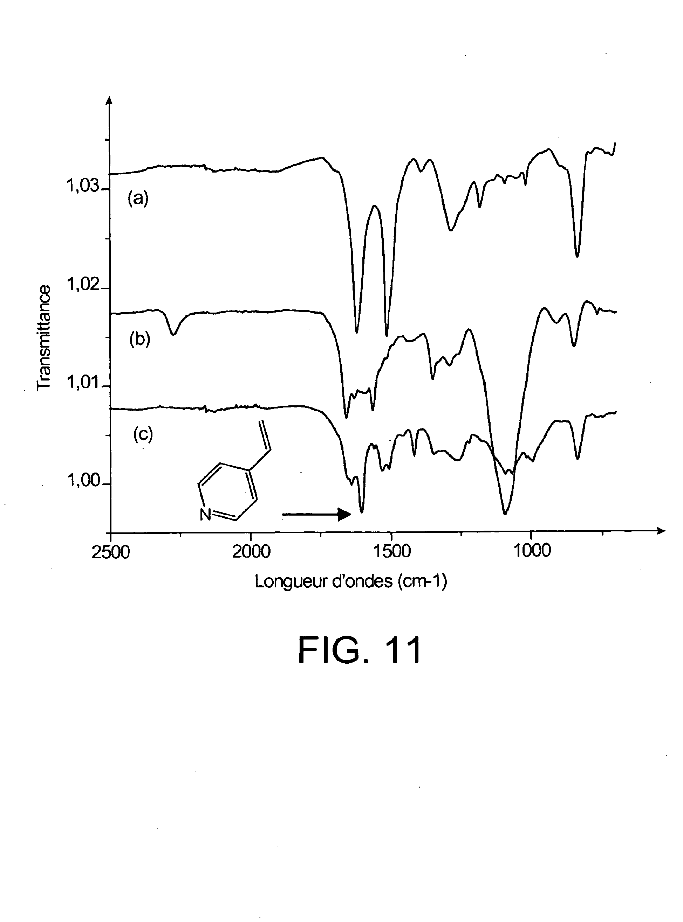

La Figure 11 présente le spectre IR d'une surface revêtue d'un précurseur de primaire de type polyaminophénylène (a), d'un primaire (b) et après réaction avec la 4-vinylpyridine (4VP) (c).

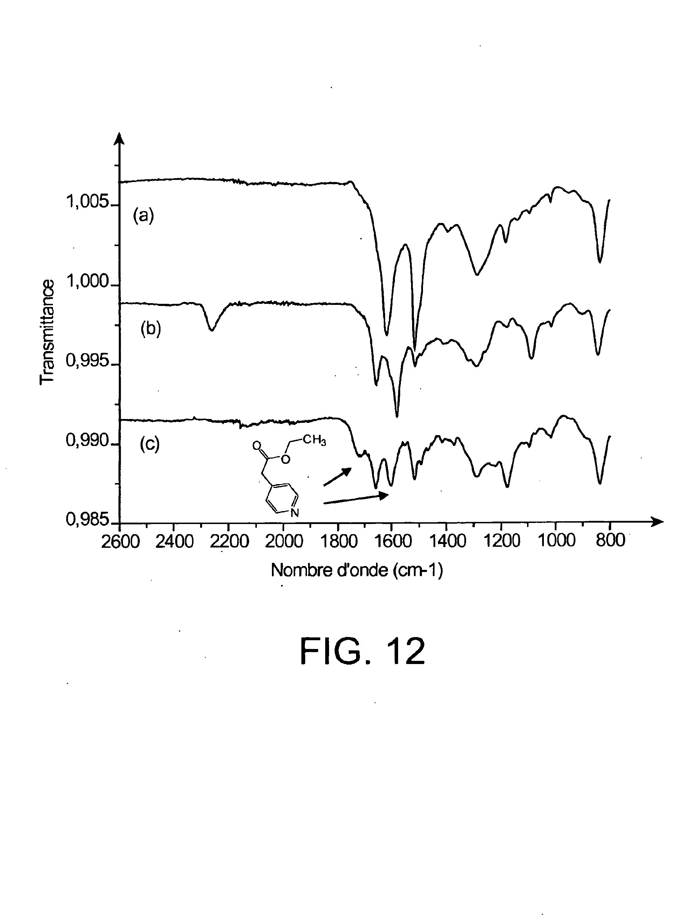

La Figure 12 présente le spectre IR d'une surface revêtue d'un précurseur de primaire de type polyaminophénylène (a), d'un primaire (b) et après réaction avec l'éthyl-4-pyridylacétate (c).
La Figure 13 présente le spectre IR d'une surface revêtue d'un précurseur de primaire de type polyaminophénylène (a), d'un primaire (b) et après réaction avec la poly-4-vinylpyridine (P4VP) (b).
La Figure 14 présente le spectre IR d'une surface revêtue d'un précurseur de primaire de type polyaminophénylène (a), d'un primaire (b) et après réaction avec le PAMAM (c).
La Figure 15 présente l'analyse par spectrométrie IR de lames d'or sur lesquelles a été greffé, par greffage chimique radicalaire, pendant 180 min, un film d'un précurseur de primaire de type phénylamine supportée (1211081), d'un primaire de type phényldiazonium supporté (1211082) et après réaction de ce primaire avec une molécule hydrophobe qu'est le Zonyl® (1211083).
La Figure 16 présente l'angle de contact mesuré (5 mesures indépendantes) pour une goutte d'eau sur des lames de verre sur lesquelles a été greffée une chaîne perfluorée issue de Zonyl® selon le procédé décrit au point III-3, une lame de verre vierge servant de contrôle.
La Figure 17 présente la photographie d'une goutte d'eau sur une lame de verre vierge (Figure 17A) et celle d'une goutte d'eau sur une lame de verre sur laquelle a été greffée une chaîne perfluorée issue de Zonyl® selon le procédé décrit au point III-3 (Figure 17B).

### EXPOSÉ DÉTAILLÉ DE MODES DE RÉALISATION PARTICULIERS

Les exemples qui suivent ont été réalisés, sauf mention contraire, dans des conditions normales de température et de pression (environ 25°C sous environ 1 atm) à l'air ambiant. Sauf mention contraire, les réactifs employés ont été directement obtenus dans le commerce sans purification supplémentaire. Les réactions ont été effectuées à l'aide de lames de verre de 7,5 x 1,2 cm recouvertes par évaporation sous vide de 5 nm de chrome puis de 200 nm d'or).

Aucune précaution n'a été prise concernant la composition de l'atmosphère et les solutions n'ont pas été dégazées.

### I - Préparation de la première surface

### I-1 Préparation du précurseur de primaire d'adhésion

### I-1-1 Protocole chimique

L'apprêt a été préparé d'après le protocole qui a été illustré dans [Chem. Mater. 2007, 19, 6323-6330].

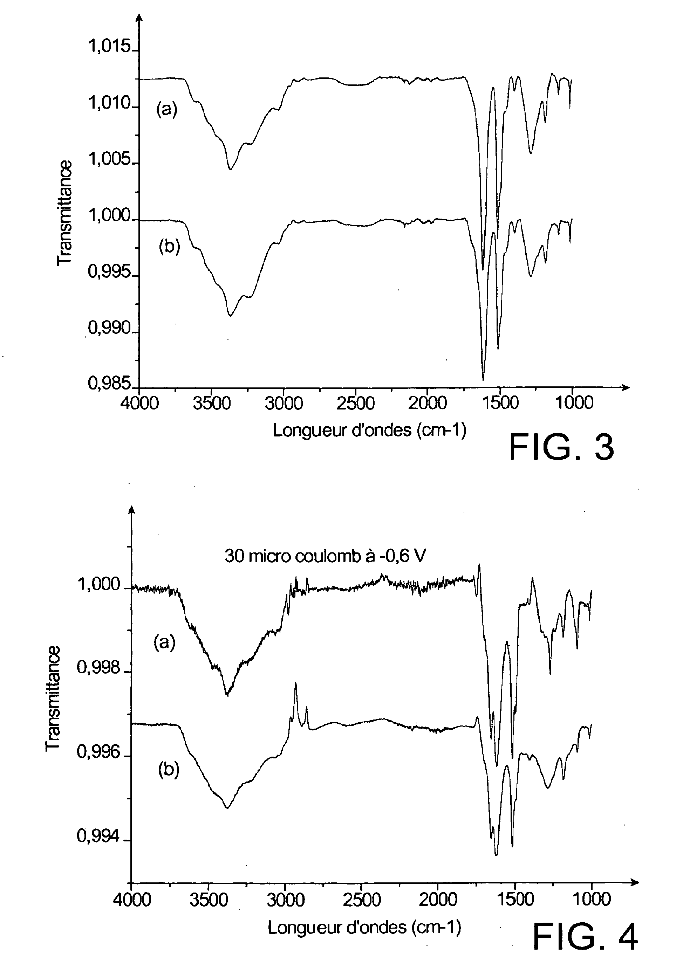

Des lames de verre ont été plongées durant 40 min dans un mélange comportant 2 ml d'une solution aqueuse de NH₂-Ph-NH₂ (5.10⁻³ M dans HCl 0,5 M), 2 ml de solution aqueuse de NaNO₂ (5.10⁻³ M) et 80 mg de limaille de fer. A la différence du protocole Chem. Mater. 2007, 19,6323-6330, la réaction est faite ici à 35 °C afin d'obtenir des films plus épais. Le spectre infrarouge (IR) de l'échantillon analysé après 40 min de réaction est montré sur la Figure 3.

La résistance aux ultrasons de la couche formée a été testée avec succès dans l'acétonitrile. Les spectres infrarouge réalisés avant et après ultrasons (respectivement spectres (a) et (b), Figure 3) sont similaires et indiquent par conséquent qu'il n'y a pas eu de perte de matière et que le film est solidement greffé.

Des bandes de membranes de Polyfluorure de vinylidène (PVDF)β (1 cm x 4 cm et 25 µm d'épaisseur) ont été plongées durant 120 min dans un mélange comportant 2 ml d'une solution aqueuse de NH₂-Ph-NH₂ (5.10⁻³ M dans HCl 0,5 M), 2 ml de solution aqueuse de NaNO₂ (5.10⁻³ M) et 80 mg de limaille de fer. A la différence du protocole Chem. Mater. 2007, 19, 6323-6330, la réaction est faite ici à 35 °C afin d'obtenir des films plus épais.

### I-1-2 Protocole électrochimique

Le protocole électrochimique qui a été employé est similaire à celui décrit dans [*Chem. Mater.* **2006,** *18*, 4755-4763]. La réaction a été réalisée dans une cellule électrochimique contenant 10 ml d'une solution électrochimique correspondant à une solution aqueuse de 1,4-phenylènediamine (10⁻² M) et de NaNO₂ (5.10⁻³ M) dans HCl 0,5 M.

Le dépôt des films a été fait de façon potentiostatique (le potentiel choisi se situant dans la barrière d'électroactivité du sel de diazonium) ou de façon potentiodynamique (voltamétrie cyclique) avec une vitesse de balayage de 20 mV.s⁻¹.

Les spectres IR des films obtenus avant et après un traitement ultrasons dans l'acétonitrile (respectivement spectres (a) et (b), Figure 4) sont similaires. Il n'y a donc pas eu de perte de matière, et le film qui a été formé est solidement greffé.

### I-2 Formation du primaire d'adhésion

Les substrats revêtus de l'apprêt (chimiques comme électrochimiques), obtenus en I-1, peuvent être traités en milieu organique comme en milieu aqueux pour créer une fonction diazonium.

### I-2-1 Milieu organique

Les surfaces revêtues de l'apprêt (forme amine aromatique d'origine chimique ou électrochimique) ont été plongées dans une solution d'acétonitrile contenant du NOBF₄ (10⁻² M) durant 30 secondes. Durant cette étape, la concentration de NOBF₄ n'a pas besoin d'être précise, il y a toujours un excès par rapport aux fonctions amines présentes à la surface.

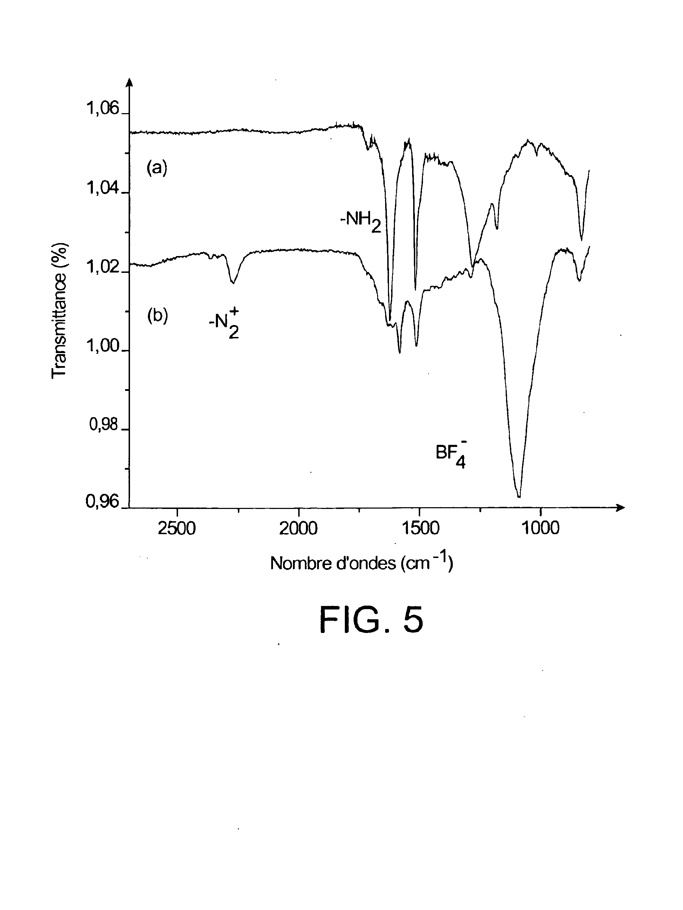

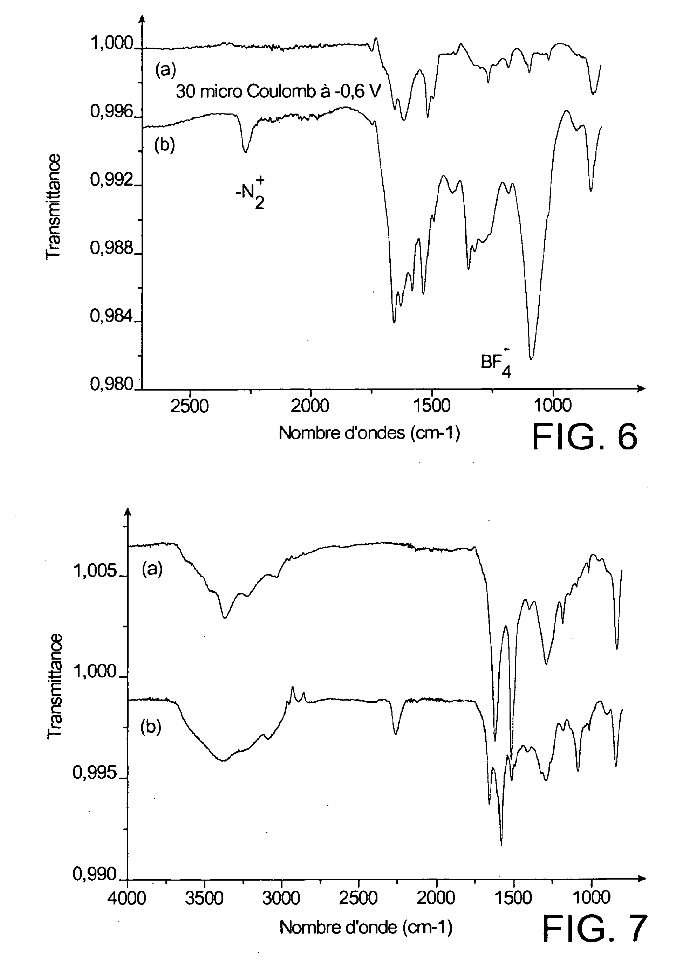

La formation des sels de diazonium a pu être suivie par spectrométrie infrarouge : un pic à 2270 cm⁻¹ correspondant au diazonium et un pic à 1080 cm⁻¹ correspond à son contre-ion BF₄⁻ apparaissent comme illustré à la figure 5 pour un film d'origine chimique (I-1-1), et figure 6 pour un film d'origine électrochimique (I-1-2).

La réaction mise en oeuvre dans l'exemple I-2-1 peut être schématisée de la façon suivante :

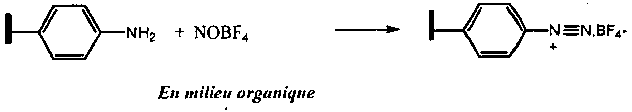

### I-2-2 Milieu aqueux

Les surfaces revêtues de l'apprêt ont été plongées dans une solution aqueuse de HCl 0,5 M et de NaNO₂ 5.10⁻² M. Durant cette étape, la concentration de NaNO₂ n'a pas besoin d'être précise, il y a toujours un excès par rapport aux fonctions amines présentes à la surface.

La formation des sels de diazonium a pu être suivie par spectrométrie IR : un pic à 2270 cm⁻¹ correspondant au diazonium comme illustré à la figure 7. La réaction mise en oeuvre dans l'exemple I-2-2 peut être schématisée de la façon suivante :

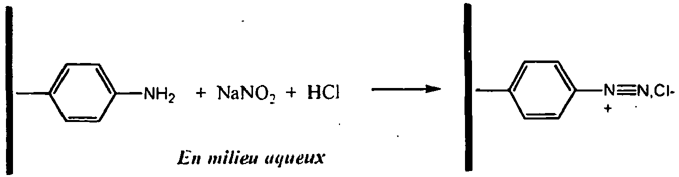

### I-3 Structuration de la surface ou « patterning »

Certaines des surfaces obtenues après les traitements décrits au paragraphe I-2 sont recouvertes d'un masque. Le masque se présente sous la forme d'une plaque de verre lithographiée avec un métal opaque. La surface possédant les motifs lithographiés est mise directement en contact avec la couche auto-adhésive activée puis l'ensemble a été soumis à une irradiation UV sous une lampe UV (spectre 300-500 nm) pendant quelques minutes.

L'apparition d'un « patterning » à la surface du support contenant des parties « mortes » et des plots auto-adhésifs activés a pu être mise en évidence par mapping en spectrométrie IR avec l'apparition d'un pic à 2270 cm⁻¹ pour les zones protégées par le masque et la disparition du pic à 2270 cm⁻¹ pour les zones irradiées aux UV.

Certaines surfaces de lames d'or sont recouvertes d'un masque réalisé à l'aide d'un marqueur tel que ceux proposés en papeterie. Après les traitements décrits au paragraphe I-2, l'apparition d'un « patterning » à la surface du support contenant des parties nues et des plots auto-adhésifs activés a pu être mise en évidence par mapping en spectrométrie IR avec l'apparition d'un pic à 2270 cm⁻¹ pour les zones non protégées par le masque et la disparition du pic à 2270 cm⁻¹ pour les zones recouvertes de marqueurs.

### II - Assemblage/Immobilisation

### II-1 Immobilisation de matériaux inorganiques

### II-1-1 Graphène par exfoliation d'HOPG

Un bloc d'HOPG (obtenu auprès de Advanced Ceramics Corporation, grade ZYH, de dimensions 12 x 12 x 2 mm) déposé sur un ruban adhésif (fourni par 3M, scotch magic^{™} 810 invisible) a été mis au contact des surfaces obtenues aux exemples I-2. Une pression, comprise entre 0,1 et 10 bar, a été exercée, à l'aide d'une presse mécanique à serrage à clef, et l'ensemble a été placé dans une étuve à 100 °C durant une heure.

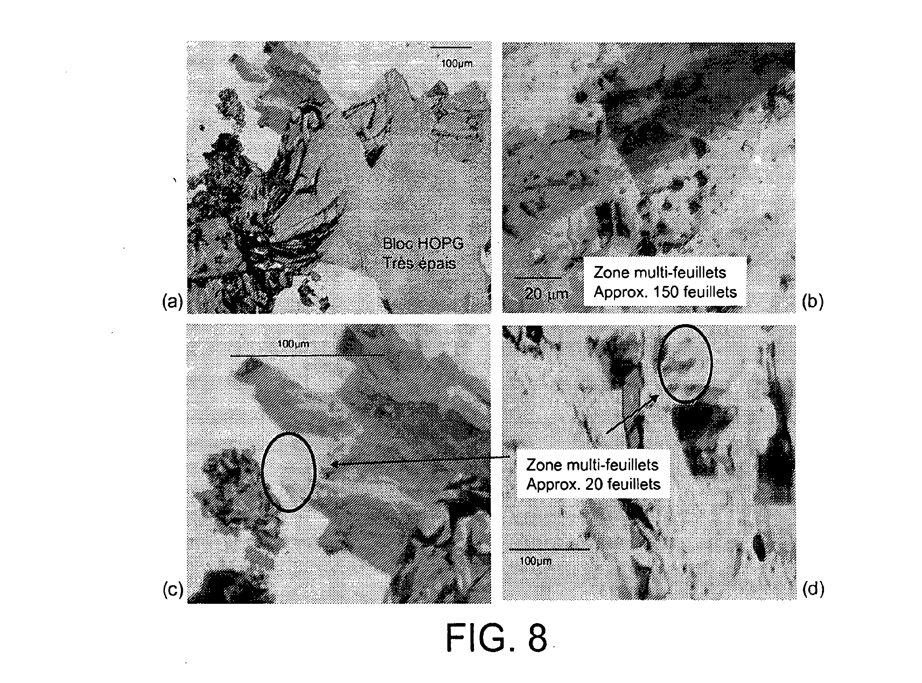

A l'issue de ce traitement, le feuillet de graphène du bloc d'HOPG directement en contact avec le primaire d'adhésion est greffé. Le bloc greffé a une épaisseur d'environ 50 nm soit environ 150 feuillets de graphène (Figure 8B).

La caractérisation exacte du mono-feuillet demande une analyse Raman difficile à réaliser sur certaines zones qui paraissent diffuses (figures 8C et 8D) : un nombre de feuillets inférieur à 20 est certainement obtenu. Le bloc d'HOPG greffé a résisté à un traitement aux ultrasons (Figure 8A).

### II-1-2 Nanotubes de carbone (CNT)

Des nanotubes de carbone multi-parois (MWCNTs) de diamètre externe compris entre 4 et 15 nm (synthétisés par CCVD, très purs et fournis pas la société Nanocyl) ont été ajoutés pour 0,3 mg/ml dans la n-méthylpyrolidone puis exposés à des ultrasons durant 6 h pour conduire à une dispersion stable.

Une centrifugation à 7000 tours/min a permis de séparer les CNT mal dispersés. Dix ml de surnageant ont été prélevés et une lame de verre dorée, préparée selon le protocole I-2, y a été immergée. Le milieu réactionnel a été maintenu à 100 °C sous agitation. Après 12 h, la lame a été retirée puis rincée abondamment avec de l'éthanol et de l'acétone.

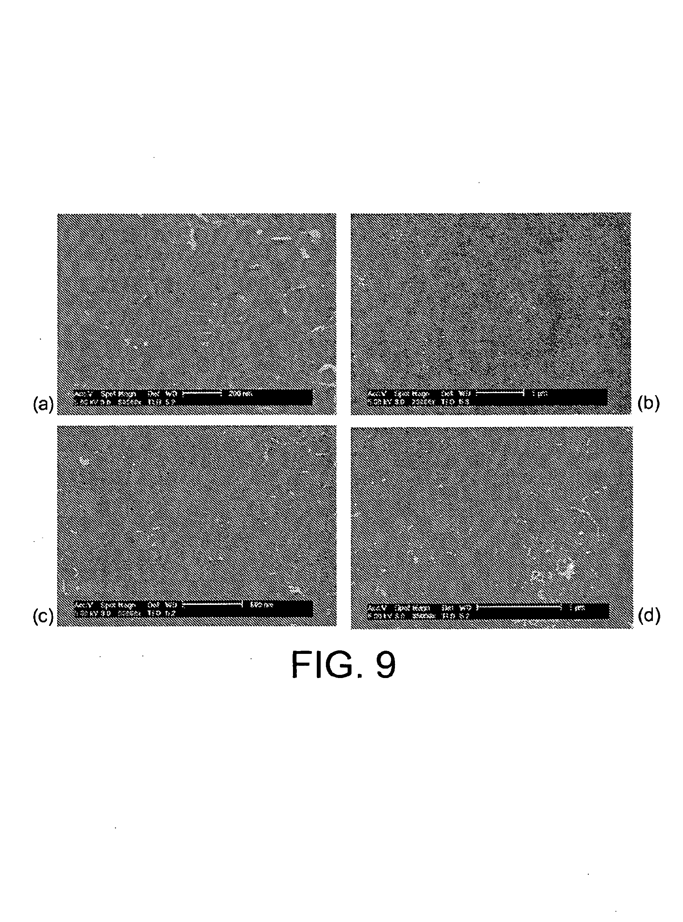

Les surfaces ainsi traitées ont été analysées par microscopie électronique à balayage, le dépôt homogène de CNT observé à différents grossissements est présenté sur les figures 9A, 9B, 9C et 9D.

### II-1-3 Nanoparticules de cuivre

Les nanoparticules de cuivre ont été préparées de la façon suivante : dans un bécher, contenant 50 ml d'une solution aqueuse de CuSO₄ (250 mg CuSO₄,5H₂ dit sulfate de cuivre pentahydrate dans 50 ml d'eau déionisée), on a ajouté 2 g de surfactant HEA₁₆Cl [chlorure de N,N-diméthyl-N-cétyl-N-(2-hydroxyéthyl) ammonium] sous agitation magnétique pendant plusieurs minutes (typiquement de 2 à 10 min). Puis ont été introduits, dans le bécher, 2 ml d'une solution de NaBH₄. La solution de NaBH₄ a été préparée en dissolvant 150 mg de NaBH₄ dans 2 ml d'eau déionisée. L'agitation a été stoppée lorsqu'une coloration bleu-noir est apparue (typiquement 1 à 5 min). Puis la solution a évolué vers une couleur rouge-noir en 5 à 10 min.

La surface revêtue par les sels de diazonium a alors été trempée dans la solution typiquement entre 5 et 30 secondes. Des temps plus longs ont permis une couverture plus complète. Les échantillons ont ensuite été rincés à l'eau déionisée. Des surfaces de référence vierges et simplement revêtues du polyphénylène amine ont aussi été trempées dans les mêmes conditions pour évaluer l'adsorption spontanée des nanoparticules sur ces surfaces.

Les images de ces différentes surfaces obtenues en microscopie électronique à balayage ont révélé la présence de cuivre dendritique seulement sur la surface revêtue par les sels de diazonium et non sur les surfaces de référence.

### II-2 Immobilisation de composés organiques

### II-2-1 Composés organiques simples : acétylpyridine, 4-vinyl pyridine, éthyl-4-pyridylacétate

Des lames de verre comportant un primaire d'adhésion préparées selon les protocoles exposés en I-2 ont été employées.

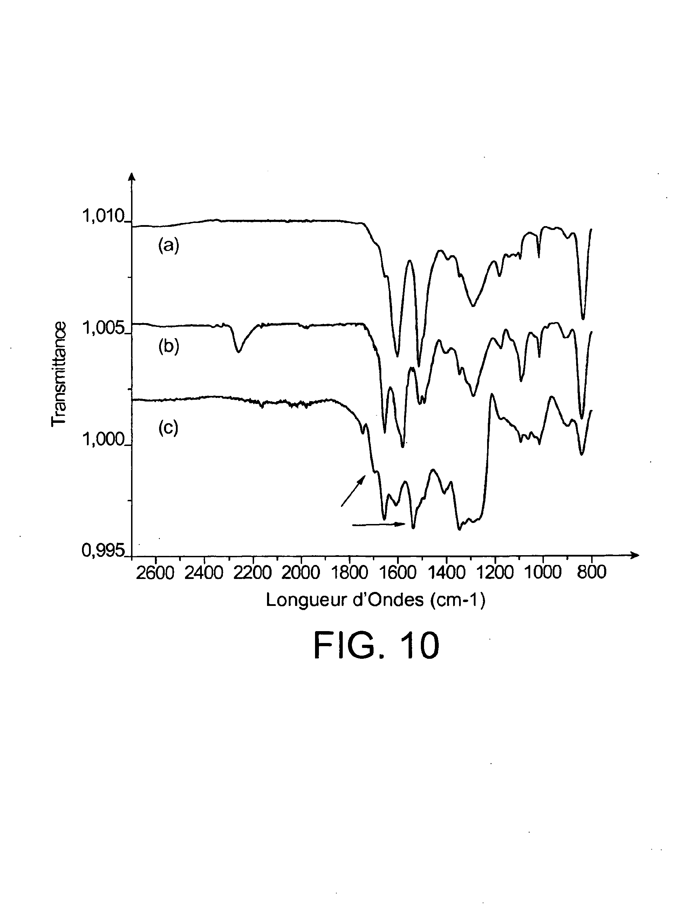

Les échantillons ont été immergés 3 min dans une solution d'acétylpyridine, de 4-vinyl pyridine ou d'éthyl-4-pyridylacetate. Après rinçage à la diméthylformamide (DMF) et exposition aux ultrasons, les surfaces ont été analysées par spectrométrie IR, les spectres sont présentés sur les figures 10 (acétylpyridine), 11 (4-vinyl pyridine) et 12 (éthyl-4-pyridylacetate).

La réaction mise en oeuvre dans l'exemple II.2.1 avec la 4-vinyl pyridine est probablement la suivante :

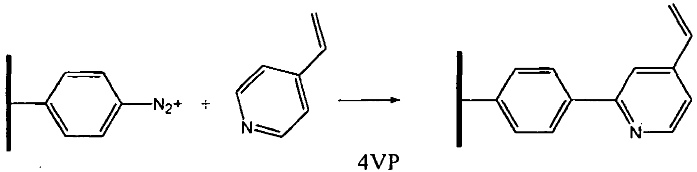

### II-2-2 Polymère : Poly-4-vinylpyridine

Des lames de verre comportant un primaire d'adhésion préparées selon les protocoles exposés en I-2 ont été employées.

La poly-4-vinylpyridine (P4VP) a été dissoute à hauteur de 2% en masse dans de la diméthyl formamide (DMF) pour réaliser une bonne dispersion. La lame de verre a été placée sur une tournette et recouverte par la solution de P4VP. Une rotation à 2000 tours/min pendant une minute a permis de former un film mince de P4VP. Cinq minutes après le dépôt, la lame a été rincée et exposée aux ultrasons dans la DMF.

La réaction mise en oeuvre dans l'exemple II.2.2 est probablement la suivante :

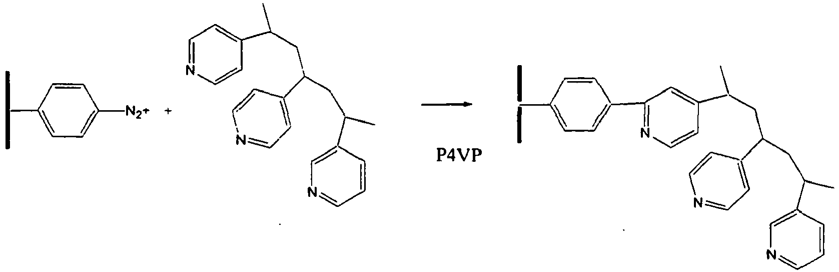

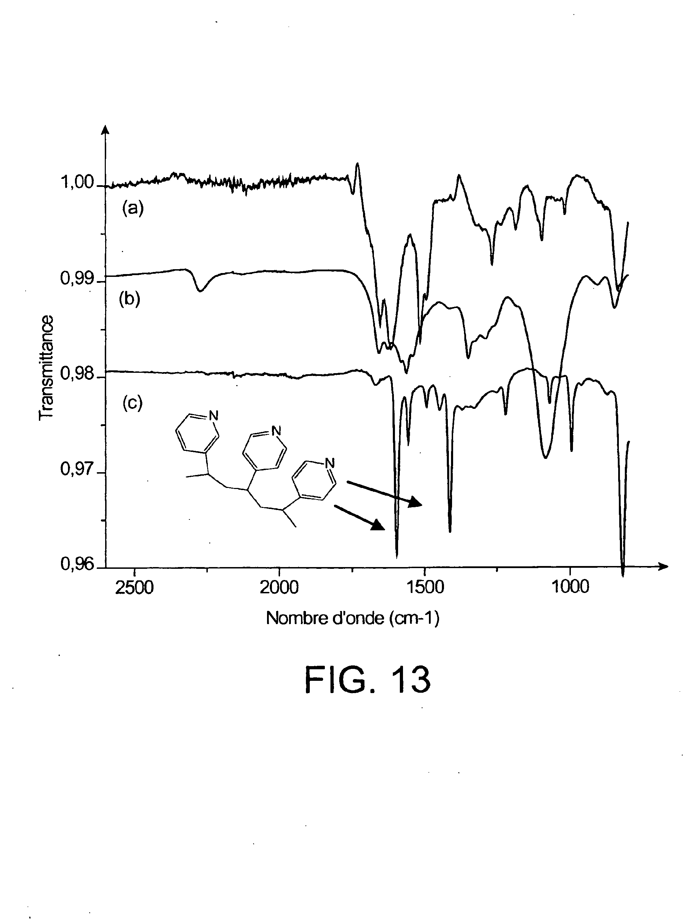

La figure 13 présente les spectres IR obtenus pour la surface primaire (a) et la surface revêtue par la P4VP (b). Les pics caractéristiques des cycles pyridines sont présents vers 1400 et 1600 cm⁻¹.

### II-2-3 Dendrimères : PAMAM

Des lames de verre comportant un primaire d'adhésion préparées selon les protocoles exposés en I-2 ont été employées.

Du PAMAM (Sigma Aldrich) de formule : 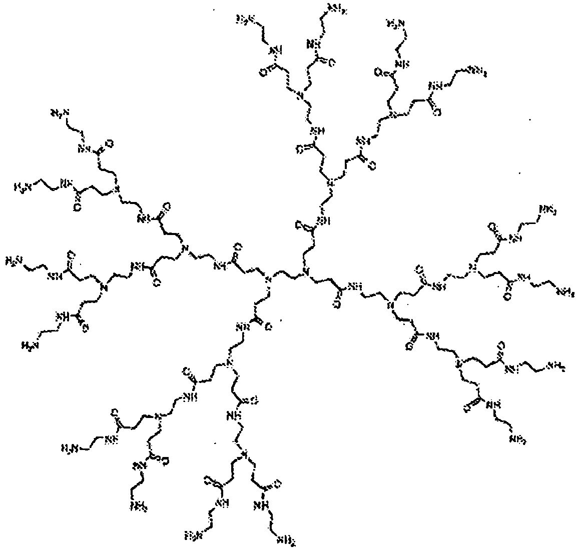 a été dissous à hauteur de 5% en masse dans de la diméthyl formamide (DMF) pour réaliser une bonne dispersion. Les lames de verre ont été placées sur une tournette et recouvertes par la solution de PAMAM. Une rotation à 2000 tours/min pendant une minute a permis de former un film mince de PAMAM. Cinq minutes après le dépôt, la lame a été rincée avec une solution de HCl (0,5 M), à la DMF puis à l'acétone.

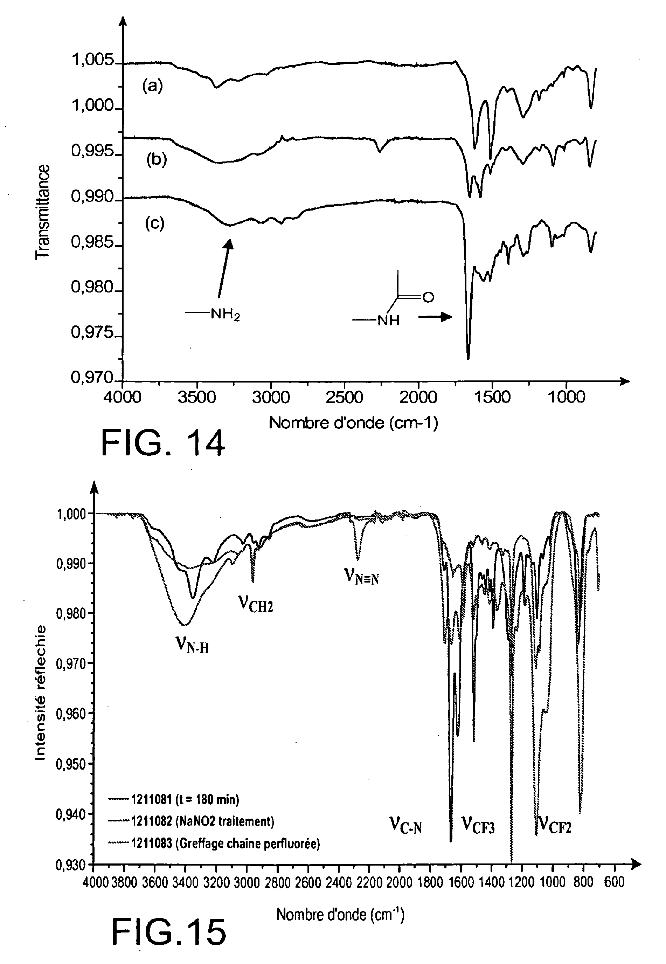

Sur les spectres IR réalisés à l'issue de la réaction sur les lames, on peut voir les pics caractéristiques du PAMAM : vers 3200 cm⁻¹ pour les aminés et 1670 cm⁻¹ pour les fonctions amides (Figure 14).

### II-3 Immobilisation de molécules biologiques

### II-3-1 Etude de la stabilité dans le temps

Des lames de verre recouvertes d'une fine couche d'or présentant une épaisseur de l'ordre de 100 nm ont été préparées en mettant en oeuvre les étapes de traitement décrites aux paragraphes I-1-1 (ou I-1-2), I-2-2 et I-3. Les lames ainsi traitées seront appelées ci-après « lames (ou surfaces) revêtues d'une couche auto-adhésive activée ».

Les lames ont été immergées dans de l'eau milliQ pH=7 puis retirées de la solution à différents temps et séchées. Ces lames ont été ensuite analysées par spectrométrie IR. L'intensité de la bande à 2270 cm⁻¹ en fonction du temps à été déterminée.

### II-3-2 Immobilisation d'un peptide synthétique linéaire déprotégé

Le peptide GPGGVVGP (SEQ ID NO: 1 dans la liste de séquences en annexe) a été synthétisé, en utilisant la stratégie Fmoc à 0,1 mmol. Une résine commerciale préchargée Fmoc-Gly-Wang (0,5 g, 0,8 mmol/g) est placée dans le réacteur du synthétiseur automatique de peptide. Les acides aminés N°-Fmoc (Gly, Pro et Val) sont utilisés avec un excès de 10 fois avec du HBTU (O-benzotriazole-N,N,N',N'-tétraméthyl-uronium-hexafluoro-phosphate) en présence de HOBt (N-hydroxybenzotriazole) et de DIEA (N, N'-diisopropyldiethylamine) pendant 16 h. On obtient 0, 368 g de peptide lié à la résine, qui est déprotégée à l'extrémité N-terminale.

Le peptide est clivé de la résine grâce à une solution de TFA/H₂O/TIS (acide trifluoroacétique/ eau/triisopropylesilane) (92 :2,5 :2,5) pendant 3 h à température ambiante. La résine est éliminée par filtration et elle est lavée avec du TFA (2*1 mL) et du dichlorométhane (10 mL). Le filtrat est évaporé sous pression réduite puis noyé dans de l'éther diéthylique froid. Après 12 h à -20 °C, un précipité (100 mg) est isolé et collecté après filtration. Après purification par chromatographie HPLC préparative, 39,5 mg de peptide pur est obtenu.

2 mg de ce peptide ont été dissous dans 1,5 mL de DMSO. 3 x 200 µL de cette solution ont été déposés sur 3 échantillons différents : sur une lame de verre recouverte d'or, sur une lame revêtue d'une couche auto-adhésive activée et sur une surface revêtue de la « couche morte ». Le dépôt à été réalisé sans précaution particulière et à température ambiante. Après 10 min de réaction, les 3 supports sont rincés avec du DMSO puis soumis à des lavages sous sonication : DMSO 2 min/éthanol 2 min/eau milliQ 2 min. La surface des trois supports est alors séchée sous N₂. Les trois supports sont analysés par spectrométrie IR. L'apparition d'une bande à 1670 cm⁻¹ (Amide I) sur le support comportant la couche auto-adhésive activée confirme la présence et le greffage covalent du peptide GPGGVVGP à la surface. La bande 1670 cm⁻¹ spécifique du peptide n'a pas été retrouvée sur le support or et sur le support « couche morte ». Ces résultats confirment bien le greffage covalent du peptide par la couche auto-adhésive.

### II-3-3 Immobilisation d'un peptide cyclique synthétique semi-protégé

Du CBO-P11 semi-protégé (SEQ ID NO: 2 dans la liste de séquences en annexe) de formule : 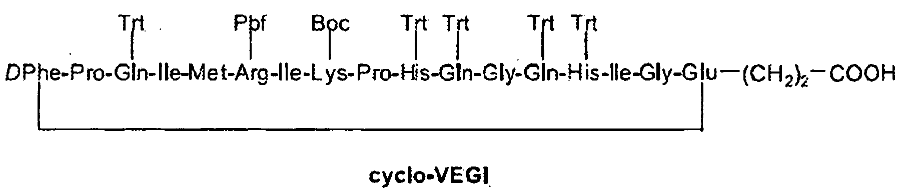 a été synthétisé selon la méthode décrite dans [Goncalves et al., 2005. Pharmaceutical Research, vol. 22, N°8, p1411-1421].

Ce peptide cyclique synthétique semi-protégé a été dissous dans du DMSO (1 mg dans 3 mL). 3 x 200 µL de cette solution ont été déposés sur 3 échantillons différents : sur une lame de verre recouverte d'or, sur une lame revêtue de la couche auto-adhésive activée et sur une surface revêtue de la « couche morte ». Le dépôt à été réalisé sans précaution particulière et à température ambiante. Après 10 min de réaction, les 3 supports sont rincés avec du DMSO puis soumis à des lavages sous sonication : DMSO 2 min/éthanol 2 min/eau milliQ 2 min. La surface des trois supports est alors séchée sous N₂. Les trois supports sont analysés par spectrométrie IR. L'apparition de bandes à 1666 cm⁻¹ (Amide I) et à 1534 cm⁻¹ (Amide II) sur le support comportant la couche auto-adhésive activée confirme la présence et le greffage covalent du peptide cyclique semi-protégé à la surface.

Les bandes à 1666 cm⁻¹ (Amide I) et à 1534 cm⁻¹ (Amide II) spécifiques du peptide ont été retrouvées sur le support or et sur le support « couche morte » avec une intensité comparable à 10% de celle mesurée sur le support comportant la couche auto-adhésive activée. Ceci est dû à l'adsorption non spécifique du peptide cyclique semi-protégé à la surface de ces supports. Le peptide adsorbé sur ces supports peut être éliminé en modifiant les conditions environnementales, à titre d'exemples, le milieu et/ou sa salinité. Cependant, ces résultats confirment bien le greffage covalent du peptide par la couche auto-adhésive.

### II-3-4 Immobilisation d'un peptide cyclique synthétique déprotégé

Du CBO-P11 (Calbiochem) de formule : cyclic(*D*-Phe-Pro-Gln-Ile-Met-Arg-Ile-Lys-Pro-His-Gln-Gly-Gln-His-Ile-Gly-Glu) (SEQ ID NO: 3 dans la liste de séquences en annexe) a été dissous dans de l'eau milliQ (1 mg dans 3 mL). 3 x 200 µL de cette solution ont été déposés sur 3 échantillons différents : sur une lame de verre recouverte d'or, sur une surface revêtue de la couche auto-adhésive activée et sur une surface revêtue de la « couche morte ». Le dépôt à été réalisé sans précaution particulière et à température ambiante. Après 10 min de réaction, les 3 supports sont rincés avec de l'eau milliQ puis soumis à un lavage sous sonication dans de l'eau milliQ pendant 2 min. La surface des trois supports est alors séchée sous N₂. Les trois supports sont analysés par spectrométrie IR.

L'apparition de bandes à 1662 cm⁻¹ (Amide I) et à 1514 cm⁻¹ (Amide II) sur le support comportant la couche auto-adhésive activée confirme la présence et le greffage covalent du peptide cyclic(D-Phe-Pro-Gln-Ile-Met-Arg-Ile-Lys-Pro-His-Gln-Gly-Gln-His-Ile-Gly-Glu) (SEQ ID NO: 3 dans la liste de séquences en annexe) à la surface. Les bandes à 1662 cm⁻¹ (Amide I) et à 1514 cm⁻¹ (Amide II) spécifiques du peptide ont été retrouvées sur le support or et sur le support « couche morte » avec une intensité comparable à 10% de celle mesurée sur le support comportant la couche auto-adhesive activée. Ceci est dû à l'adsorption non spécifique du CBO-P11 à la surface des supports. Le peptide adsorbé sur ces supports peut être éliminé en modifiant les conditions environnementales, à titre d'exemples, le milieu et/ou sa salinité. Cependant, ces résultats confirment bien le greffage covalent du peptide par la couche auto-adhésive.

### II-3-5 Immobilisation de mono-amino- β-cyclodextrine

La mono-amino-β-cyclodextrine a été synthétisée selon la procédure [Baugh et al., 2001. J. Am. Soc. Chem., vol. 123 (50), 12488-12494]. 2 mg de mono-amino-β-cyclodextrine ont été dissous dans 2 ml d'eau milliQ. 3 x 200 µL de cette solution ont été déposés sur 3 échantillons différents : sur une lame de verre recouverte d'or, sur une surface revêtue de la couche auto-adhésive activée et sur une surface revêtue de la « couche morte ». Le dépôt à été réalisé sans précaution particulière et à température ambiante. Après 10 min de réaction, les 3 supports sont rincés avec de l'eau milliQ puis soumis à des lavages sous sonication : eau milliQ 2 min/éthanol 2 min/eau milliQ 2 min. La surface des trois supports est alors séchée sous N₂. Les trois supports sont analysés par spectrométrie IR.

L'apparition de bandes à 1023, 1153, 2037 et à 3321 cm⁻¹ sur le support comportant la couche auto-adhésive activée confirme la présence et le greffage covalent de la mono-amino-β-cyclodextrine à la surface. Les bandes 1023, 1153, 2037 et à 3321 cm⁻¹ spécifiques de la β-cyclodextrine n'ont pas été retrouvées sur le support or et sur le support « couche morte ». Ces résultats confirment bien le greffage covalent de la mono-amino-β-cyclodextrine par la couche auto-adhésive.

### II-3-6 Immobilisation d'ADN

De l'ADN de faible poids moléculaire de sperme de Saumon (Fluka) a été dissous dans de l'eau milliQ (1 mg dans 3 mL). 3 x 200 µL de cette solution ont été déposés sur 3 échantillons différents : sur une lame de verre recouverte d'or, sur une surface revêtue de la couche auto-adhésive activée et sur une surface revêtue de la « couche morte ». Le dépôt à été réalisé sans précaution particulière et à température ambiante. Après 10 min de réaction, les 3 supports sont rincés avec de l'eau milliQ puis soumis à des lavages sous sonication : eau milliQ 2 min/éthanol 2 min/eau milliQ 2 min. La surface des trois supports est alors séchée sous N₂. Les trois supports sont analysés par spectrométrie IR.

L'apparition de bandes à 1226 cm⁻¹ et à 1080 cm⁻¹ sur le support comportant la couche auto-adhésive activée confirme la présence et le greffage covalent d'ADN à la surface. Les bandes à 1226 cm⁻¹ et à 1080 cm⁻¹ spécifiques de l'ADN n'ont pas été retrouvées sur le support or et sur le support « couche morte ». Ces résultats confirment bien le greffage covalent de l'ADN par la couche auto-adhésive.

### II-3-7 Immobilisation de la Glucose Oxydase sur lame de verre recouverte d'or

De la glucose oxydase (Sigma Aldrich) a été dissoute dans de l'eau milliQ (1 mg dans 3 mL). 3 x 200 µL de cette solution ont été déposés sur 3 échantillons différents : sur une lame de verre recouverte d'or, sur une surface revêtue de la couche auto-adhésive activée et sur une surface revêtue de la « couche morte ». Le dépôt à été réalisé sans précaution particulière et à température ambiante. Après 10 min de réaction, les 3 supports sont rincés avec de l'eau milliQ puis soumis à des lavages sous sonication : eau milliQ 2 min/éthanol 2 min/eau milliQ 2 min. La surface des trois supports est alors séchée sous N₂. Les trois supports sont analysés par spectrométrie IR.

L'apparition de bandes à 1659 cm⁻¹ (Amide I), à 1546 cm⁻¹ (Amide II) et 1255 cm⁻¹ (Amide III) sur le support comportant la couche auto-adhésive activée confirme la présence et le greffage covalent de la glucose oxydase à la surface. Les bandes à 1659 cm⁻¹ (Amide I), à 1546 cm⁻¹ (Amide II) et à 1255 cm⁻¹ (Amide III) spécifiques de la glucose oxydase n'ont pas été retrouvées sur le support or et sur le support « couche morte ». Ces résultats confirment bien le greffage covalent de la glucose oxydase par la couche auto-adhésive.

Un support présentant la glucose oxydase obtenue comme décrit ci-dessus a été plongé dans un réacteur contenant une solution de tampon PBS à pH = 7 de volume égal à 13 mL. Le support a été relié à un potentiostat. Le système comprend une contre-électrode (composée d'une plaque de graphite) et une électrode de référence du type ECS (pour « Electrode au Calomel Saturé »). L'électrode a subi un balayage de potentiel de type voltamétrie cyclique à la vitesse de 20 mV.s⁻¹ entre le potentiel d'équilibre (typiquement observé entre 0,1 et 0,3 V) et + 0,750 mV au balayage aller puis un balayage retour jusqu'à - 0,2 V et enfin un retour au potentiel d'équilibre où la tension a été interrompue. Les valeurs limites de potentiel de la voltamétrie cyclique ont été déterminées par l'ajout contrôlé d'H₂O₂ (et en absence de la glucose oxydase) dans le milieu afin de déterminer la zone d'électroactivité de l'H₂O₂. Une première mesure de courant a été réalisée sans glucose et a permis d'obtenir la référence du système. Après l'ajout de glucose, on mesure le courant à - 0,200 mV et à + 0,500 mV. La variation de courant a été suivie au cours du temps. Trois concentrations différentes de glucose ont été utilisées : 7,69.10⁻⁴ M et 7,69.10⁻³ M.

Il apparaît très clairement que l'apparition de H₂O₂ en fonction du temps suit bien une loi du type hyperbolique croissante compatible avec la relation de Mickaelis-Menten. Lorsque la solution contenant le glucose a été remplacée par une solution de tampon PBS, une hyperbole décroissante est apparue au cours du temps jusqu'à l'obtention d'un spectre de voltamétrie identique à celui de référence.

| [Glc]= 7,69.10⁻⁴ M | 1 min | 7 min | 20 min | 40 min | 60 min |
|---|---|---|---|---|---|
| Δ I (a.u.) | 0,39 | 1, 05 | 6,82 | 18,68 | 31,38 |

Pour une concentration en glucose de 7,69.10⁻⁴ M, la vitesse initiale de la réaction est de 0,4916 a.u./min.

| [Glc]= 7,69.10⁻³ M | 11 min | 31 min | 41 min | 51 min | 61 min | 81 min |
|---|---|---|---|---|---|---|
| Δ I (a.u.) | 10,18 | 20,98 | 29,48 | 35,68 | 38,88 | 42,38 |

Pour une concentration en glucose de 7,69.10⁻³ M, la vitesse initiale de la réaction est de 0,9255 a.u./min.

En traçant le graphe en double inverse 1/vitesse initiale = f(1/[glucose]), une constante de Michaelis Kₘ observée a été déterminée de l'ordre de 113 mM. Ce résultat est en accord avec les valeurs de Kₘ de la glucose oxydase trouvées dans la littérature (Km = 33-115 mM).

### II-3-8 Immobilisation de la Glucose Oxydase sur du polyfluorure de vinylidène (PVDF)

Des bandes de PVDF β ont été préparées en mettant en oeuvre les étapes de traitement décrits aux paragraphes I-1-1 (ou I-1-2), I-2-2 et I-3. De la glucose oxydase (Sigma Aldrich) a été dissoute dans de l'eau milliQ (1 mg dans 3 mL). 2 x 200 µL de cette solution ont été déposés sur 2 échantillons différents : sur une bande de PVDF β vierge et sur une surface revêtue de la couche auto-adhésive activée. Le dépôt a été réalisé sans précaution particulière et à température ambiante. Après 10 min de réaction, les 2 supports sont rincés avec de l'eau milliQ puis soumis à des lavages sous sonication : eau milliQ 2 min/éthanol 2 min/eau milliQ 2 min. La surface des deux supports est alors séchée sous N₂. Les deux supports sont analysés par spectrométrie IR.

L'apparition d'une bande à 1659 cm⁻¹ (Amide I), sur le support comportant la couche auto-adhésive activée confirme la présence et le greffage covalent de la glucose oxydase à la surface. La bande à 1659 cm⁻¹ (Amide I) spécifique de la glucose oxydase n'a pas été retrouvée sur la bande de PVDF β vierge. Ces résultats confirment bien le greffage covalent de la glucose oxydase par la couche auto-adhésive.

### III - Utilisation du procédé selon l'invention pour modifier l'énergie de surface d'une surface

### III-1 Stratégie de greffage mise en oeuvre

La synthèse est réalisée en trois étapes.

Dans la première étape, on greffe le 1,4-aminophényldiazonium (obtenu *in situ* par oxydation de la 1,4-diaminophénylène par le nitrite de sodium). Dans la seconde étape, les fonctions amine greffées sont diazotisées par action du nitrite de sodium. Dans la dernière, la surface comprenant maintenant le sel de diazonium est mise en contact (40°C< T <70°C, UV, réducteur) avec une molécule hydrophobe (Zonyl®) et la surface modifiée, portant des molécules hydrophobes qui lui sont chimiquement liées, est obtenue.

### III-2 Réactifs

Les réactifs mis en oeuvre dans cet exemple sont les suivants :
- du 1,4-diaminophenylène : F.W. = 108,14 ; m = 0,324 g ; n = 3 mmoles ; 1 eq.
- du nitrite de sodium : F.W. = 68,995 ; m = 0,207 g ; n = 3 mmoles ; 1 eq.
- du HCl : F.W. = 36,46 ; C = 4 M ; v = 20 mL.
- de l'H₂O : v = 15 mL.
- de la poudre de fer : F.W. = 55,85 ; m = 1,0 g; n = 18 mmoles ; 1 eq.
- du Zonyl® : F.W. = 443 ; d = 1,17 ; v = 2 mL ; n = 7,7 mmoles; 1 eq.

### III-3 Protocole

**Etape 1 :** Dans un bécher de 50 mL, le 1,4-diaminophenylène (0,324 g, 3.10⁻³ mol) a été solubilisé dans une solution d'acide chlorhydrique (20 mL de 4 M) sous agitation magnétique à température ambiante. A cette solution jaune pâle, 15 mL d'une solution aqueuse de nitrite de sodium (0,207 g, 3.10⁻³ mol) ont été ajoutés doucement pour donner une solution rouge bordeaux. Deux lames en verre ainsi qu'une lame d'or utilisées comme référence pour vérifier par IR l'efficacité du greffage ont été ensuite immergées dans le bain. La poudre de fer (1,0 g, 18.10⁻³ mol) a été ensuite versée. Les lames de verre et d'or ont été retirées au bout de 180 min puis rincées successivement à l'eau MQ, éthanol, acétone et plongées dans un bain de DMF à 60°C pendant 15 min avant de commencer les analyses IR.

**Etape 2 :** Les lames précédemment greffées ont été immergées dans une solution de nitrite de sodium à 0,1 M (25 mL) diluée dans 25 mL d'une solution de HCl 0,5 M pendant 1 min. Ces lames sont rincées à l'eau puis séchées sous flux d'argon avant les analyses IR.

**Etape 3 :** Les lames sont enfin plongées dans le Zonyl® à 35°C pendant 1h, rincées et analysées par IR.

### III-4 Résultats

L'analyse par spectrométrie IR des lames d'or après l'étape 1 confirme la présence du film attendu (Figure 15, 1211081). Les bandes spécifiques à 3342 cm⁻¹ (déformation NH), 1665 cm⁻¹ et 1616 cm⁻¹ (déformation C-N) sont visibles. Une estimation des épaisseurs (% de greffage) de revêtement est obtenue par la mesure du pourcentage d'absorption de la bande la plus intense du spectre, ici la C-N à 1616 cm⁻¹.

Les conversions des fonctions amines en diazoniums (étape 2) puis des diazoniums en éther fluorés (étape 3) sont également confirmées par IR (bandes à 2268 cm⁻¹ pour le N=N ; 1103 et 1263 cm⁻¹ pour les CF₃ et CF₂) (Figure 15, 1211082 et 1211083).

### Fichiers :

1211081 (t = 180 min, or) 6,1% de greffage
1211082 (Traitement NaNO₂)
1211083 (Greffage chaîne perfluorée).

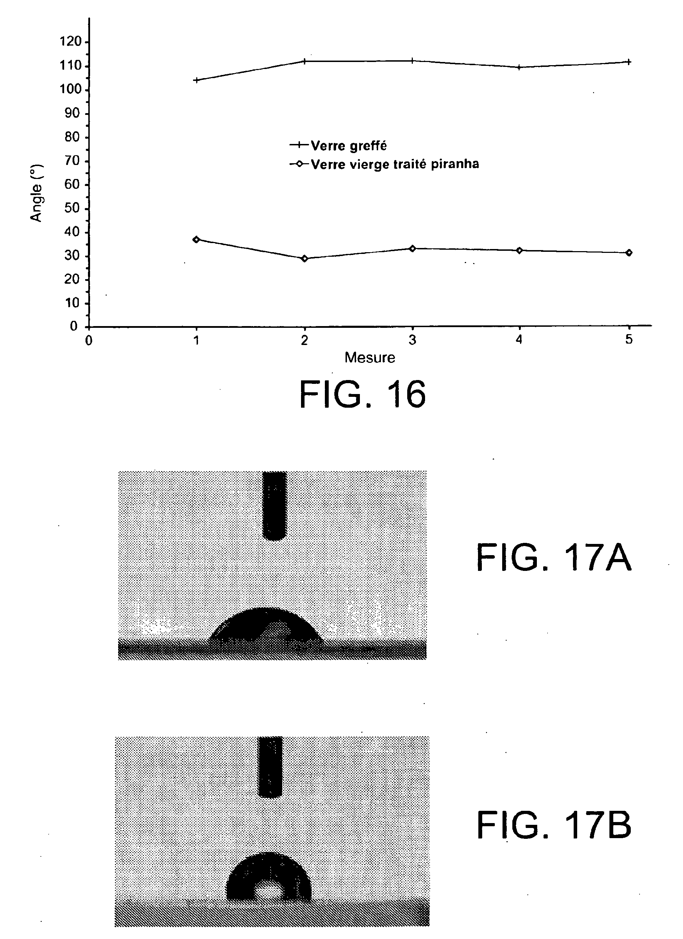

La figure 16 présente les valeurs de l'angle de contact obtenu pour une goutte d'eau disposée sur une lame de verre vierge ou sur une lame de verre sur laquelle a été greffée une chaîne perfluorée issue de Zonyl® selon le procédé décrit au point III-3 (5 mesures indépendantes). La Figure 17 est une photographie de cette goutte sur une lame de verre vierge (Figure 17A) ou sur une lame de verre ainsi greffée (Figure 17B).

### SEQUENCE LISTING

<110> Commissariat à l'Energie Atomique
<120> Procédé pour assembler deux surfaces ou une surface avec une molécule
   d'intérêt.
<130> SP32960 PCT
<140> PCT/EP2009/XXXX
   <141> 2009-04-02
<150> FR08/52209
   <151> 2008-04-03
<150> FR08/57269
   <151> 2008-10-27
<160> 3
<170> PatentIn version 3.1
<210> 1
   <211> 8
   <212> PRT
   <213> Artificial
<220>
   <223> Peptide synthétique linéaire déprotégé
<400> 1
   Gly Pro Gly Gly Val Val Gly Pro
   1               5
<210> 2
   <211> 17
   <212> PRT
   <213> Artificial
<220>
   <223> Peptide cyclique synthétique semi-protégé (CBO-P11)
<220>
   <221> MISC_FEATURE
   <222> (1)..(1)
   <223> D-phénylalanine
<220>
   <221> CHAIN
   <222> (3)..(3)
   <223> Glutamine portant un groupement protecteur trityle
<220>
   <221> CHAIN
   <222> (6)..(6)
   <223> Arginine portant un groupement protecteur 2,2,4,6,7-pentamethyldi hydrobenzofuran-5-sulfonyle (Pbf)
<220>
   <221> CHAIN
   <222> (8)..(8)
   <223> Lysine portant un groupement protecteur t-butoxycarbonyle (Boc)

## Revendications

1. Procédé d'assemblage d'au moins une zone d'une première surface avec au moins une zone d'une seconde surface ou avec une molécule d'intérêt, **caractérisé en ce que** ledit procédé comprend les étapes successives suivantes :
a) éventuellement soumettre une zone de ladite première surface présentant au moins un précurseur d'un primaire d'adhésion à des conditions adéquates permettant d'obtenir, à partir d'un précurseur d'un primaire d'adhésion, au moins un primaire d'adhésion,
b) soumettre ladite zone de ladite première surface présentant au moins un primaire d'adhésion éventuellement obtenu à l'étape (a) à des conditions non-électrochimiques pour obtenir, sur ladite zone, au moins une entité radicalaire ;
c) mettre en contact ladite zone de ladite première surface présentant au moins une entité radicalaire obtenue à l'étape (b), avec ladite zone de ladite seconde surface ou avec ladite molécule d'intérêt,
dans laquelle ladite première surface portant au moins un primaire d'adhésion est de formule (I) suivante :
(première surface) - (B)ₙ-R-N₂⁺, A⁻ (I)
dans laquelle :
- (B)ₙ représente un agent de liaison,
- n est égal à 0 ou 1,
- A représente un anion monovalent et
- R représente un groupe aryle.

2. Procédé d'assemblage selon la revendication 1, **caractérisé en ce que** ledit agent de liaison B se présente sous forme d'un polymère ou copolymère, issu de plusieurs unités monomériques d'espèces chimiques identiques ou différentes.

3. Procédé d'assemblage selon l'une quelconque des revendications 1 ou 2, **caractérisé en ce que** la molécule d'intérêt est choisie dans le groupe constitué par les molécules organiques comportant des bases organiques faibles, les macromolécules organiques, les molécules biologiques et les molécules hydrophobes.

4. Procédé d'assemblage selon l'une quelconque des revendications 1 à 3, **caractérisé en ce que** lesdites première et seconde surfaces sont constituées d'un matériau identique ou différent, choisi dans le groupe constitué par les métaux, les alliages métalliques, le bois, le papier, le coton, le feutre de carbone, le silicium, les nanotubes, les matériaux graphitiques, les matériaux organiques, les polymères fluorés ou non fluorés et le diamant,
ladite seconde surface présentant éventuellement au moins un atome pouvant être impliqué dans une réaction chimique radicalaire et/ou ionique.

5. Procédé d'assemblage selon l'une quelconque des revendications précédentes, **caractérisé en ce que** ledit primaire d'adhésion est lié de manière directe à ladite zone de ladite première surface.

6. Procédé d'assemblage selon l'une quelconque des revendications précédentes, **caractérisé en ce que** ledit primaire d'adhésion est lié de manière indirecte à ladite zone de ladite première surface.

7. Procédé d'assemblage selon l'une quelconque des revendications précédentes, **caractérisé en ce que** ledit primaire d'adhésion est un sel d'aryle clivable choisi dans le groupe constitué par le tétrafluoroborate de phényldiazonium supporté, le tétrafluoroborate de 4-nitrophényldiazonium supporté, le tétrafluoroborate de 4-bromophényldiazonium supporté, le chlorure de 4-aminophényldiazonium supporté, le chlorure de 2-méthyl-4-chlorophényldiazonium supporté, le tétrafluoroborate de 4-benzoylbenzènediazonium supporté, le tétrafluoroborate de 4-cyanophényldiazonium supporté, le tétrafluoroborate du 4-carboxyphényldiazonium supporté, le tétrafluoroborate de 4-acétamidophényldiazonium supporté, le tétrafluoroborate de l'acide 4-phénylacétique diazonium supporté, le sulfate de 2-méthyl-4-[(2-méthylphényl)diazényl]benzènediazonium supporté, le chlorure de 9,10-dioxo-9,10-dihydro-1-anthracènediazonium supporté, le tétrafluoroborate de 4-nitronaphtalènediazonium supporté et le tétrafluoroborate de naphtalènediazonium supporté, ledit groupe aryle étant, de préférence, choisi parmi les groupes aryles substitués par des groupements attracteurs d'électrons.

8. Procédé d'assemblage selon l'une quelconque des revendications précédentes, **caractérisé en ce que** lesdites conditions non-électrochimiques sont choisies dans le groupe constitué par les conditions thermiques, cinétiques, chimiques, photochimiques, radiochimiques et leurs combinaisons.

9. Procédé d'assemblage selon l'une quelconque des revendications précédentes, **caractérisé en ce que** ladite mise en contact est effectuée de façon directe ou en solution.

10. Procédé d'assemblage selon l'une quelconque des revendications précédentes, **caractérisé en ce que** lesdites étapes (b) et (c) sont réalisées de façon simultanée.

11. Procédé d'assemblage selon l'une quelconque des revendications précédentes, **caractérisé en ce que** ladite zone de ladite première surface présentant au moins un précurseur d'un primaire d'adhésion est une zone de ladite première surface à laquelle est lié, de façon covalente, un film organique présentant au moins un précurseur d'un primaire d'adhésion, avantageusement choisi dans le groupe constitué par la phénylamine supportée, la 4-nitrophénylamine supportée, la 4-bromophénylamine supportée, la 4-aminophénylamine supportée, la 2-méthyl-4-chlorophénylamine supportée, la 4-benzoylbenzèneamine supportée, la 4-cyanophénylamine supportée, la 4-carboxyphénylamine supportée, la 4-acétamidophénylamine supportée, l'acide 4-amino benzoïque supporté, la 2-méthyl-4-[(2-méthylphényl)diazényl]amine supportée, la 9,10-dioxo-9,10-dihydro-1-anthracèneamine supportée, la 4-nitronaphtalèneamine supportée et la naphtalèneamine supportée.

12. Support solide dont la surface présente au moins une zone avec au moins un primaire d'adhésion, tel que défini à l'une quelconque des revendications 1, 2, 4 à 7 ou 11.

13. Utilisation d'un support solide tel que défini à la revendication 12 pour préparer une biopuce ou un biocapteur.

14. Biopuce ou biocapteur comprenant un support solide dont la surface présente au moins une zone avec au moins une entité radicalaire et/ou ionique telle que définie à l'une quelconque des revendications 1, 2 ou 4 ayant réagi avec un élément biologique choisi dans le groupe constitué par les peptides, les protéines, les anticorps et fragments d'anticorps, les récepteurs cellulaires ou membranaires, les polysaccharides, des cellules ou parties cellulaires et les acides nucléiques.

15. Kit d'éléments susceptible d'être utilisé lors de la mise en oeuvre d'un procédé d'assemblage tel que défini à l'une quelconque des revendications 1 à 11, comprenant :
- dans un premier compartiment, un support solide tel que défini à la revendication 12,
- éventuellement, dans un second compartiment, au moins un élément nécessaire pour élaborer une espèce radicalaire à partir du primaire d'adhésion,
- éventuellement, dans un troisième compartiment, un autre support solide ou un nano-objet dont la surface correspond à la seconde surface telle que définie à la revendication 4, ou une molécule d'intérêt telle que définie à la revendication 3, à immobiliser.

16. Utilisation d'un support solide tel que défini à la revendication 12 pour immobiliser sur ce dernier un autre support solide ou un nano-objet dont la surface correspond à la seconde surface telle que définie à la revendication 4, ou une molécule d'intérêt telle que définie à la revendication 3.

17. Utilisation d'un procédé d'assemblage tel que défini à l'une quelconque des revendications 1 à 11 ou d'un support solide tel que défini à la revendication 12 pour exfolier des feuillets de graphène.

18. Procédé pour modifier l'énergie de surface d'au moins une surface d'un solide consistant à assembler ladite surface avec une molécule hydrophobe selon un procédé d' assemblage tel que défini à l'une quelconque des revendications 1 à 3 et 5 à 11,
ledit procédé comprenant éventuellement une étape supplémentaire, suite audit assemblage, consistant à soumettre la surface assemblée avec ladite molécule à un traitement thermique.

## Patentansprüche

1. Verfahren zum Verbinden von zumindest einem Bereich einer ersten Oberfläche mit zumindest einem Bereich einer zweiten Oberfläche bzw. mit einem interessierenden Molekül, **dadurch gekennzeichnet, dass** das Verfahren die nachstehenden, aufeinanderfolgenden Schritte umfasst:
a) gegebenenfalls Aussetzen eines Bereichs der ersten Oberfläche, der zumindest einen Vorläufer eines Haftprimers aufweist, adäquaten Bedingungen, die gestatten, ausgehend von einem Vorläufer eines Haftprimers zumindest einen Haftprimer zu erhalten,
b) Aussetzen des Bereichs der ersten Oberfläche, der zumindest einen gegebenenfalls in Schritt (a) gewonnenen Haftprimer aufweist, nicht-elektrochemischen Bedingungen, um an dem Bereich zumindest eine radikale Einheit zu gewinnen,
c) Inkontaktbringen des Bereichs der ersten Oberfläche, der zumindest eine in Schritt (b) gewonnene radikale Einheit aufweist, mit dem Bereich der zweiten Oberfläche bzw. mit dem interessierenden Molekül,
wobei die erste Oberfläche, die zumindest einen Haftprimer trägt, die nachfolgende Formel (I) hat:
(erste Oberfläche) - (B)ₙ - R - N₂*, A- (I)
worin:
- (B)ₙ ein Bindemittel darstellt,
- n gleich 0 bzw. 1 ist,
- A ein einwertiges Anion darstellt und
- R eine Aryl-Gruppe darstellt.

2. Verbindungsverfahren nach Anspruch 1, **dadurch gekennzeichnet, dass** das Bindemittel B in Form eines Polymers bzw. Copolymers vorliegt, das aus mehreren Monomereinheiten gleicher oder unterschiedlicher chemischer Spezies stammt.

3. Verbindungsverfahren nach einem der Ansprüche 1 oder 2, **dadurch gekennzeichnet, dass** das interessierende Molekül ausgewählt ist aus der Gruppe umfassend organische Moleküle mit schwachen, organischen Basen, organische Makromoleküle, biologische Moleküle und hydrophobe Moleküle.

4. Verbindungsverfahren nach einem der Ansprüche 1 bis 3, **dadurch gekennzeichnet, dass** die erste und die zweite Oberfläche aus einem gleichen bzw. unterschiedlichen Material bestehen, das ausgewählt ist aus der Gruppe umfassend Metalle, Metalllegierungen, Holz, Papier, Baumwolle, Kohlenstofffilz, Silicium, Nanoröhren, Graphitmaterialien, organische Materialien, Fluorpolymere, nicht fluorierte Polymere und Diamant,
wobei die zweite Oberfläche gegebenenfalls zumindest ein Atom aufweist, das bei einer radikalen und/oder ionischen chemischen Reaktion beteiligt ist.

5. Verbindungsverfahren nach einem der vorangehenden Ansprüche, **dadurch gekennzeichnet, dass** der erste Haftprimer in direkter Weise mit dem Bereich der ersten Oberfläche verbunden ist.

6. Verbindungsverfahren nach einem der vorangehenden Ansprüche, **dadurch gekennzeichnet, dass** der erste Flaftprimer in indirekter Weise mit dem Bereich der ersten Oberfläche verbunden ist.

7. Verbindungsverfahren nach einem der vorangehenden Ansprüche, **dadurch gekennzeichnet, dass** der Haftprimer ein spaltbares Arylsalz ist, ausgewählt aus der Gruppe umfassend geträgertes Phenyldiazoniumtetrafluorborat, geträgertes 4-Nitrophenyldiazoniumtetrafluorborat, geträgertes 4-Bromphenyldiazoniumtetrafluorborat, geträgertes 4-Aminophenyldiazoniumchlorid, geträgertes 2-Methyl-4-chlorphenyldiazoniumchlorid, geträgertes 4-Benzoylbenzoldiazoniumtetrafluorborat, geträgertes 4-Cyanphenyldiazoniumtetrafluorborat, geträgertes 4-Carboxyphenyldiazoniumtetrafluorborat, geträgertes 4-Acetamidophenyl-diazoniumtetrafluorborat, geträgertes 4-Phenylessigsäurediazoniumtetrafluorborat, geträgertes 2-Methyl-4-[(2-methylphenyl)diazenyl]-benzoldiazoniumsulfat, geträgertes 9,10-Dioxo-9,10-dihydro-1-anthracendiazoniumchlorid, geträgertes 4-Nitronaphthalindiazoniumtetrafluorborat und geträgertes Naphthalindiazoniumtetrafluorborat,
wobei die Arylgruppe vorzugsweise ausgewählt ist aus Arylgruppen, die durch elektronenziehende Gruppen substituiert sind.

8. Verbindungsverfahren nach einem der vorangehenden Ansprüche, **dadurch gekennzeichnet, dass** die nicht-elektrochemischen Bedingungen ausgewählt sind aus der Gruppe umfassend thermische, kinetische, chemische, photochemische, radiochemische Bedingungen und deren Kombinationen.

9. Verbindungsverfahren nach einem der vorangehenden Ansprüche, **dadurch gekennzeichnet, dass** das Inkontaktbringen direkt oder in Lösung erfolgt.

10. Verbindungsverfahren nach einem der vorangehenden Ansprüche, **dadurch gekennzeichnet, dass** die Schritte (b) und (c) gleichzeitig durchgeführt werden.

11. Verbindungsverfahren nach einem der vorangehenden Ansprüche, **dadurch gekennzeichnet, dass** der Bereich der ersten Oberfläche, der zumindest einen Vorläufer eines Haftprimers aufweist, ein Bereich der ersten Oberfläche ist, mit dem in kovalenter Weise ein organischer Film verbunden ist, der zumindest einen Vorläufer eines Haftprimers aufweist, vorteilhaft ausgewählt aus der Gruppe umfassend geträgertes Phenylamin, geträgertes 4-Nitrophenylamin, geträgertes 4-Bromophenylamin, geträgertes 4-Aminophenylamin, geträgertes 2-Methyl-4-Chlorophenylamin, geträgertes 4-Benzoylbenzolamin, geträgertes 4-Cyanophenylamin, geträgertes 4-Carboxyphenylamin, geträgertes 4-Acetamidophenylamin, geträgerte 4-Aminobenzoesäure, geträgertes 2-Methyl-[(2-methylphenyl]diazenyl]-Amin, geträgertes 9,10-Dioxo-9,10-dihydro-1-anthracenamin, geträgertes, 4-Nitronaphthalinamin und geträgertes Naphthalinamin.

12. Fester Träger, dessen Oberfläche zumindest einen Bereich mit zumindest einem Haftprimer aufweist, wie in einem der Ansprüche 1, 2 4 bis 7 oder 11 definiert.

13. Verwendung eines festen Trägers, wie in Anspruch 12 definiert, zum Herstellen eines Biochips bzw. eines Biosensors.

14. Biochip bzw. Biosensor mit einem festen Träger, dessen Oberfläche zumindest einen Bereich mit zumindest einer radikalen und/oder ionischen Einheit wie in einem der Ansprüche 1, 2 oder 4 definiert aufweist, der mit einem biologischen Element reagiert hat, das ausgewählt ist aus der Gruppe umfassend Peptide, Proteine, Antikörper und Antikörper-Fragmente, zelluläre oder membrangebundene Rezeptoren, Polysaccharide, Zellen oder Zellenteile und Nukleinsäuren.

15. Teilesatz, der beim Durchführen eines Verbindungsverfahrens wie in einem der Ansprüche 1 bis 11 definiert verwendet werden kann, enthaltend:
- in einem ersten Fach einen festen Träger wie in Anspruch 12 definiert,
- gegebenenfalls in einem zweiten Fach zumindest ein Element, das erforderlich ist, um eine radikale Spezies ausgehend von dem Haftprimer herzustellen,
- gegebenenfalls in einem dritten Fach einen weiteren festen Träger oder ein Nanoobjekt, dessen Oberfläche der zweiten Oberfläche wie in Anspruch 4 definiert entspricht, oder ein zu mobilisierendes, interessierendes Molekül wie in Anspruch 3 definiert.

16. Verwendung eines festen Trägers wie in Anspruch 12 definiert, um an diesen einen weiteren festen Träger oder ein Nanoobjekt zu immobilisieren, dessen Oberfläche der zweiten Oberfläche wie in Anspruch 4 definiert oder einem interessierenden Molekül wie in Anspruch 3 definiert entspricht.

17. Verwendung eines Verbindungsverfahrens wie in einem der Ansprüche 1 bis 11 definiert bzw. eines festen Trägers wie in Anspruch 12 definiert, zur Exfoliation von Graphen-Blättern.

18. Verfahren zum Ändern der Oberflächenenergie zumindest einer Oberfläche eines Feststoffs, das darin besteht, die Oberfläche mit einem hydrophoben Molekül nach einem Verbindungsverfahren wie in einem der Ansprüche 1 bis 3 und 5 bis 11 definiert zu verbinden,
wobei das Verfahren gegebenenfalls einen zusätzlichen Schritt nach der Verbindung umfasst, der darin besteht, die mit dem Molekül verbundene Oberfläche einer Wärmebehandlung auszusetzen.

## Claims

1. Process for assembling at least one zone of a first surface with at least one zone of a second surface or with a molecule of interest, **characterized in that** said process comprises the following successive steps:
a) optionally subjecting a zone of said first surface bearing at least one adhesion primer precursor to conditions suitable for obtaining, from an adhesion primer precursor, at least one adhesion primer,
b) subjecting said zone of said first surface bearing at least one adhesion primer optionally obtained in step (a) to non-electrochemical conditions to obtain, on said zone, at least one radical species;
c) placing said zone of said first surface bearing at least one radical species, obtained in step (b), in contact with said zone of said second surface or with said molecule of interest,
wherein said first surface bearing at least one adhesion primer is of formula (I) below:
(first surface)-(B)ₙ-R-N₂⁺, A⁻ (I)
in which :
B represents a bonding agent,
n is equal to 0 or 1,
A represents a monovalent anion, and
R represents an aryl group.

2. Assembling process according to claim 1, **characterized in that** said bonding agent B is in the form of a polymer or copolymer, derived from several monomer units of identical or different chemical species.

3. Assembling process according to either of claims 1 or 2, **characterized in that** the molecule of interest is chosen from the group consisting of organic molecules comprising weak organic bases, organic macromolecules, biological molecules and hydrophobic molecules.

4. Assembling process according to any one of claims 1 to 3, **characterized in that** said first and second surfaces are constituted of an identical or different material, chosen from the group consisting of metals, metal alloys, wood, paper, cotton, carbon felt, silicon, nanotubes, graphite materials, organic materials, fluorinated or non-fluorinated polymers, and diamond,
said second surface optionally bearing at least one atom that can be involved in a radical and/or ionic chemical reaction.

5. Assembling process according to any one of preceding claims, **characterized in that** said adhesion primer is directly bonded to said zone of said first surface.

6. Assembling process according to any one of preceding claims, **characterized in that** said adhesion primer is indirectly bonded to said zone of said first surface.

7. Assembling process according to any one of preceding claims, **characterized in that** said adhesion primer is a cleavable aryl salt chosen from the group consisting of supported phenyldiazonium tetrafluoroborate, supported 4-nitrophenyldiazonium tetrafluoroborate, supported 4-bromophenyldiazonium tetrafluoroborate, supported 4-aminophenyldiazonium chloride, supported 2-methyl-4-chlorophenyldiazonium chloride, supported 4-benzoylbenzenediazonium tetrafluoroborate, supported 4-cyanophenyldiazonium tetrafluoroborate, supported 4-carboxyphenyldiazonium tetrafluoroborate, supported 4-acetamidophenyldiazo ammonium tetrafluoroborate, supported 4-phenylacetic acid diazonium tetrafluoroborate, supported 2-methyl-4-[(2-methylphenyl)diazenyl]benzenediazonium sulfate, supported 9,10-dioxo-9,10-dihydro-1-anthracenediazonium chloride, supported 4-nitronaphthalenediazonium tetrafluoroborate and supported naphthalenediazonium tetrafluoroborate,
said aryl group being preferably chosen from aryl groups substituted with electron-withdrawing groups.

8. Assembling process according to any one of preceding claims, **characterized in that** said non-electrochemical conditions are chosen from the group consisting of thermal, kinetic, chemical, photochemical and radiochemical conditions, and combinations thereof.

9. Assembling process according to any one of preceding claims, **characterized in that** said placing in contact is performed directly or in solution.

10. Assembling process according to any one of preceding claims, **characterized in that** said steps (b) and (c) are performed simultaneously.

11. Assembling process according to any one of preceding claims, **characterized in that** said zone of said first surface bearing at least one adhesion primer precursor is a zone of said first surface to which is covalently bonded an organic film bearing at least one adhesion primer precursor, advantageously chosen from the group consisting of supported phenylamine, supported 4-nitrophenylamine, supported 4-bromophenylamine, supported 4-amino phenylamine, supported 2-methyl-4-chlorophenylamine, supported 4-benzylbenzeneamine, supported 4-cyanophenylamine, supported 4-carboxyphenylamine, supported 4-acetamidophenylamine, supported 4-aminobenzoic acid, supported 2-methyl-4-[(2-methylphenyl)diazenyl]amine, supported 9,10-dioxo-9,10-dihydro-1-anthraceneamine, supported 4-nitronaphthaleneamine and supported naphthaleneamine.

12. Solid support whose surface bears at least one zone with at least one adhesion primer as defined in any one of claims 1, 2, 4 to 7, or 11.

13. Use of a solid support as defined in claim 12, for preparing a biochip or a biosensor.

14. Biochip or biosensor comprising a solid support whose surface bears at least one zone with at least one radical and/or ionic species as defined in any one of claims 1, 2 or 4 that has reacted with a biological component chosen from the group consisting of peptides, proteins, antibodies and antibody fragments, cell or membrane receptors, polysaccharides, cells or cell parts, and nucleic acids.

15. Kit of parts that can be used during the implementation of an assembling process as defined in any one of claims 1 to 11, comprising:
- in a first compartment, a solid support as defined in claim 12,
- optionally, in a second compartment, at least one component required for producing a radical species from the adhesion primer,
- optionally, in a third compartment, another solid support or a nano-object whose surface corresponds to the second surface as defined in claim 4, or a molecule of interest as defined in claim 3, to be immobilized.

16. Use of a solid support as defined in claim 12, for immobilizing thereon another solid support or a nano-object whose surface corresponds to the second surface as defined in claim 4, or a molecule of interest as defined in claim 3.

17. Use of an assembling process as defined in any one of claims 1 to 11 or of a solid support as defined in claim 21, for exfoliating graphene flakes.

18. Process for modifying the surface energy of at least one surface of a solid, which consists in assembling said surface with a hydrophobic molecule according to an assembling process as defined in any one of claims 1 to 3 and 5 to 11,
said process optionally comprising an additional step, following said assembling, which consists in subjecting the surface assembled with said molecule to a heat treatment.
